# EUROPEAN PATENT APPLICATION

(11) **EP 3 939 664 A1**
(43) Date of publication of application: **19.01.2022**
(21) Application number: 20382647.4
(22) Date of filing: 17.07.2020
(51) Int. Cl.: A61P 31/14, A61K 31/47, C07D 215/52

(54) **COMPOUNDS FOR USE IN THE TREATMENT OF VIRAL INFECTIONS BY VIRUS OF THE FAMILY CORONAVIRIDAE**

(71) Applicant: Consejo Superior De Investigaciones Científicas, 28006 Madrid (ES)
(72) Inventor: GUTIÉRREZ RODRÍGUEZ, Marta, E - 28006 Madrid (ES); IZQUIERDO GARCÍA, Carolina, E - 28006 Madrid (ES); GASTAMINZA LANDART, Pablo, E - 28006 Madrid (ES); GARAIGORTA DE DIOS, Urtzi, E - 28006 Madrid (ES)
(74) Representative: ABG Intellectual Property Law, S.L.

(57) **Abstract**

The present invention relates to new derivatives of 2-phenyl-quinoline-4-carboxylic acid or pharmaceutically acceptable salt thereof and to combination of said compounds with other active ingredients, for use in the treatment and/or prevention of viral infections by virus from the family *Coronaviridae,* to the use of said compound or its combinations in the manufacture of a medicament for the treatment or prevention of said diseases and to a method of treating and/or preventing said diseases by administration of said compound or its combinations.

## Description

### FIELD OF THE INVENTION

The present invention relates to compounds for use in the treatment and/or prevention of viral infections by virus of the family *Coronaviridae,* especially by respiratory syndrome-related coronavirus selected from the species *"Middle East respiratory syndrome-related coronavirus"* (such as MERS-CoV) and *"Severe respiratory syndrome-related coronavirus"* (such as SARS-CoV and SARS-CoV-2).

### BAKGROUND OF THE INVENTION

The virus of the family *Coronaviridae* are enveloped viruses with a positive-sense single-stranded RNA genome and a nucleocapsid of helical symmetry. The name of the family is derived from the Latin corona, meaning "crown" or "halo", which refers to the characteristic appearance reminiscent of a solar corona around the virions (virus particles) when viewed under two-dimensional transmission electron microscopy, due to the surface being covered in club-shaped protein spikes.

In December 2019 a new infectious disease caused by severe acute respiratory syndrome coronavirus 2 (SARS-CoV-2) was first identified in Wuhan, the capital of China's Hubei province, and has since spread globally, resulting in the ongoing 2019-20 coronavirus pandemic. The disease has been given the name Coronavirus disease 2019 or COVID-19. COVID-19 has evolved into an appalling global pandemic worldwide.

The etiological agent of this severe respiratory syndrome is caused by a coronavirus, named SARS-CoV-2 due to its genetic similarity to the Severe Acute Respiratory Syndrome coronavirus (SARS-CoV) that emerged in 2003 as a limited epidemic in different areas of southeast Asia.

SARS-CoV-2 replicates efficiently in asymptomatic patients, which are largely responsible for the spread of the virus worldwide, given that high replication levels are not necessarily associated with clinical manifestations of the disease that may restrict infected patient mobility. This characteristic and the prolonged periods of time of productive infection have definitely contributed to the establishment of the current pandemic. While silent in a majority of cases, SARS-CoV-2 may lead to COVID-19, which is primarily but not exclusively characterized by severe pneumonia, that may cause death in fragile segments of the population, notably, but not exclusively in the elderly.

Common symptoms include fever, cough, and shortness of breath. Other symptoms may include muscle pain, sputum production, diarrhea, sore throat, loss of smell, and abdominal pain. While the majority of cases result in mild symptoms, some progress to bilateral pneumonia and multi-organ failure.

Early immunological studies as well as prior experience with SARS-CoV suggest that a prophylactic vaccine may be developed against SARS-CoV-2. Given the severity and wide impact of the disease, numerous efforts are being put into the development of such vaccine. While this is certainly the best approach to contain future outbreaks, it is clear that additional strategies need to be in place for patients that already acquired the infection. Antiviral treatment may contribute to infection resolution and better prognosis of hospitalized patients. Eventually, antivirals may also be considered under certain circumstances as prophylactic treatment option for the containment of specific outbreaks.

Considering that the only approved antiviral drug against SARS-CoV-2, remdesivir, is non-specific, injectable and has limited efficacy at improving COVID-19 clinical outcome, there is an urgent need for the identification of novel antivirals against SARS-CoV-2.

Given the urgency of the matter, a large effort has been done in drug repurposing, with a long list of repurposing candidates currently under clinical evaluation.

In addition to this urgent effort, it is of capital importance the discovery of new antivirals, which could allow the identification of clinical candidates. This should be the way to arrive in medium/long term to efficient drugs for COVID-19 treatment, and potentially also for other coronaviruses infections that could emerge.

### DESCRIPTION OF THE FIGURES

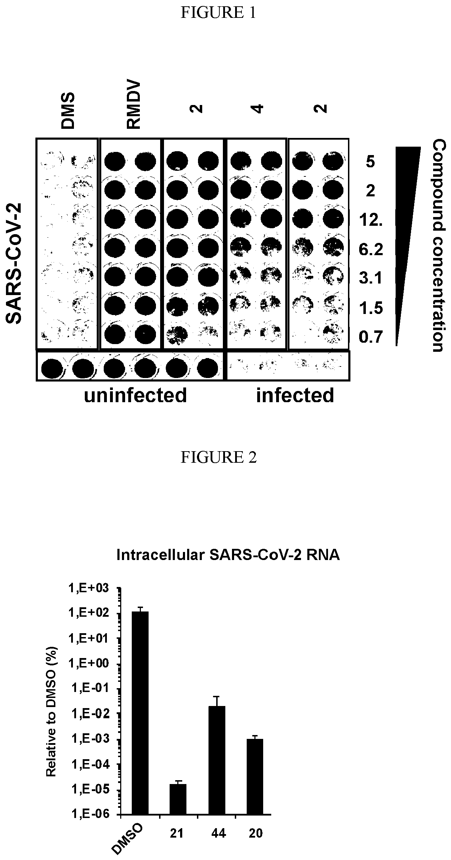

Figure 1: Cell monolayer protection assay. Figure shows a photograph of crystal violet stained Vero-E6 culture plates uninfected or infected with SARS-CoV-2 at MOI 0.001. Infected cells are dead and do not retain staining, as compared with uninfected cells that appear strongly stained. The rest of the plate was infected at MOI 0.001 although in the presence of increasing concentrations (0.78-50µM) of remdesivir (RMDVIR) and the indicated compounds or equivalent volumes of vehicle (DMSO). Except for DMSO, all tested compounds shown in the figure display protective activity against SARS-CoV-2. Compound 21 did so with comparable efficacy to remdesivir.
Figure 2 shows the quantity of viral RNA detected by qPCR when cells were treated with the compounds of examples 20, 21 and 44 in comparison with the quantity detected when the cells where only treated with the vehicle (DMSO). The qPCR results indicate that the compounds of examples 21, 44 and 20 are particularly potent antivirals at 15µM, reaching virtually undetectable levels as compared with the uninfected controls.

### SUMMARY OF THE INVENTION

The inventors have surprisingly found that certain derivatives of 2-phenylquinoline-4-carboxylic acid are useful in the treatment and/or prevention of viral infections by virus from the family *Coronaviridae.* Some of these derivatives are also new compounds not previously described in the state of the art.
Thus, in a first aspect, the present invention relates to compounds of formula (I) 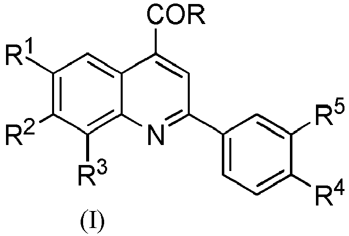 for use in the treatment and/or prevention of an infection by a virus of the family *Coronaviridae,* wherein
- R is a group selected from hydroxyl, OR' and NR'R" wherein R' and R" independently represent a group selected from allyl, benzyl, C₁₋₆ alkyl and -(O-CH₂-CH₂)ₙ-OCH₃, wherein n is an integer from 1 to 25,
- R¹, R² and R³ independently represent a hydrogen atom, a phenyl or a 5-6 membered, monocyclic, heteroaromatic ring comprising 1, 2 or 3 heteroatoms selected from N, O and S, which are optionally substituted by 1, 2 or 3 groups selected from the group consisting of C₁₋₄ alkyl, C₁₋₄ alkoxy, halogen atoms, acetyl, allylamino, morpholino and trifluoromethoxy,
- R⁴ is hydrogen, methoxy, phenyl or a 5-6 membered, monocyclic, heteroaromatic ring comprising 1, 2 or 3 heteroatoms selected from N, O and S, which are optionally substituted by 1 or 2 groups selected from the group consisting of C₁₋₄ alkyl, halogen atoms, acetyl and trifluoromethoxy, and
- R⁵ is hydrogen, halogen, phenyl or a 5-6 membered, monocyclic, heteroaromatic ring comprising 1, 2 or 3 heteroatoms selected from N, O and S, which are optionally substituted by 1, 2 or 3 groups selected from the group consisting of halogen atoms, C₁₋₄ alkyl, C₁₋₄ alkoxy, acetyl, morpholino and trifluoromethoxy,
with the condition that at least one of R¹, R², R³, R⁴ and R⁵, is not hydrogen and pharmaceutically acceptable salts thereof.

In a second aspect, the invention relates to the use of a compound as defined in the first aspect in the manufacture of a medicament for the treatment and/or prevention of viral infections by a virus of the family *Coronaviridae,* especially by respiratory syndrome-related coronaviruses selected from the species *"Middle East respiratory syndrome-related coronavirus"* (such as MERS-CoV) and *"Severe respiratory syndrome-related coronavirus"* (such as SARS-CoV and SARS-CoV-2).

In a third aspect, the invention also relates to a method of treating and/or preventing viral infections by a virus of the family *Coronaviridae,* especially by respiratory syndrome-related coronaviruses selected from the species *"Middle East respiratory syndrome-related coronavirus"* (such MERS-CoV) and *"Severe respiratory syndrome-related coronavirus"* (such as SARS-CoV and SARS-CoV-2), in a subject, comprising administering to said subject a therapeutically effective amount of a compound as defined in the first aspect of the invention.

In a fourth aspect, the present invention relates to a subgroup of new compounds selected from the group of compounds defined in the first aspect of the invention. Said subgroup is defined by formula I' wherein 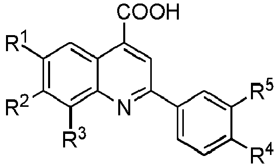
R¹ is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
R² is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
R³ is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
R⁴ is hydrogen, methoxy or phenyl optionally substituted by 1 or 2 groups selected from the group consisting of C₁₋₄ alkyl, acetyl, halogen atoms and trifluoromethoxy,
R⁵ is hydrogen, halogen or phenyl optionally substituted by 1 group selected from the group consisting of halogen atoms, C₁₋₄ alkyl, acetyl and trifluoromethoxy,
with the proviso that at least one of R¹ and R² is a phenyl group optionally substituted by 1, 2 or 3 groups selected from the group consisting of C₁₋₄ alkyl, C₁₋₄ alkoxy, halogen atoms, acetyl, morpholino and trifluoromethoxy,
and the further proviso that the compound is neither 2-(4-methoxy-phenyl)-quinoline-4-carboxylic acid nor (2,7-diphenyl-quinolin-4-carboxylic acid)
and pharmaceutically acceptable salts thereof.

In a fifth aspect, the present invention relates to a combination comprising one or more compounds as defined in the fourth aspect of the invention and or more additional compounds useful in the treatment of viral infections by virus of the family *Coronaviridae.* Some compounds that have been disclosed as potentially useful are remdesivir, ribavirin, favipiravir, chloroquine, hydroxychloroquine, lopinavir, ritonavir, umifenovir and recombinant type I interferon.

In sixth aspect, the present invention relates to a pharmaceutical composition comprising a compound of formula (I) and at least one pharmaceutically acceptable excipient for use in the treatment and/or prevention of viral infections by virus of the family *Coronaviridae.*

### DESCRIPTION OF THE INVENTION

In the first aspect, the present invention relates to a compound of formula (I) 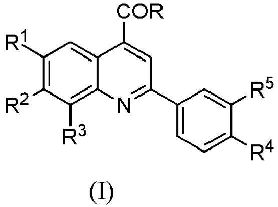 for use in the treatment and/or prevention of an infection by a virus of the family *Coronaviridae,* wherein
- R is a group selected from hydroxyl, OR' and NR'R" wherein R' and R" independently represent a group selected from allyl, benzyl, C₁₋₆ alkyl and -(O-CH₂-CH₂)ₙ-OCH₃, wherein n is an integer from 1 to 25,
- R¹, R² and R³ independently represent a hydrogen atom, a phenyl or a 5-6 membered, monocyclic, heteroaromatic ring comprising 1, 2 or 3 heteroatoms selected from N, O and S, which are optionally substituted by 1, 2 or 3 groups selected from the group consisting of C₁₋₄ alkyl, C₁₋₄ alkoxy, halogen atoms, acetyl, allylamino, morpholino and trifluoromethoxy,
- R⁴ is hydrogen, methoxy, phenyl or a 5-6 membered, monocyclic, heteroaromatic ring comprising 1, 2 or 3 heteroatoms selected from N, O and S, which are optionally substituted by 1 or 2 groups selected from the group consisting of C₁₋₄ alkyl, halogen atoms, acetyl and trifluoromethoxy, and
- R⁵ is hydrogen, halogen, phenyl or a 5-6 membered, monocyclic, heteroaromatic ring comprising 1, 2 or 3 heteroatoms selected from N, O and S, which are optionally substituted by 1, 2 or 3 groups selected from the group consisting of halogen atoms, C₁₋₄ alkyl, C₁₋₄ alkoxy, acetyl, morpholino and trifluoromethoxy,
with the condition that at least one of R¹, R², R³, R⁴ and R⁵, is not hydrogen
and pharmaceutically acceptable salts thereof.

The invention also relates to the use of a compound of formula (I) in the manufacture of a medicament for the treatment and/or prevention of viral infections by a virus of the family *Coronaviridae,* especially by respiratory syndrome-related coronaviruses selected from the species *"Middle East respiratory syndrome-related coronavirus"* (such as MERS-CoV) and *"Severe respiratory syndrome-related coronavirus"* (such SARS-CoV and SARS-CoV-2).

The invention also relates to a method of treating and/or preventing viral infections by a virus of the family *Coronaviridae,* especially by respiratory syndrome-related coronaviruses selected from the species *"Middle East respiratory syndrome-related coronavirus"* (such as MERS-CoV) and *"Severe respiratory syndrome-related coronavirus"* (such as SARS-CoV and SARS-CoV-2) in a subject, comprising administering to said subject a therapeutically effective amount of a compound of formula (I).

The term "virus of the family *Coronaviridae",* as used herein, is used to designate any viral species of the taxonomic family *"Coronaviridae"* including those of the genera *"Alphaletovirus", "Alphacoronavirus", "Betacoronavirus", "Gammacoronavirus"* and *"Deltacoronavirus" .*

In a preferred embodiment of the present invention, the virus are selected from betacoronaviruses, in particular from the lineage *"Sarbecovirus"* and still more preferably from the species *"Middle East respiratory syndrome-related coronavirus"* (such as MERS-CoV) and *"Severe respiratory syndrome-related coronavirus"* (such as SARS-CoV and SARS-CoV-2).

The terms "treating" and "treatment", as used herein, means reversing, alleviating, inhibiting the progress of, the disease or condition to which such term applies, or one or more symptoms of such disease or condition, such as lowering the viral load in a patient with respect to pretreatment levels.

The terms "preventing" and "prevention", as used herein, means avoiding or inhibiting the onset of one or more symptoms of coronavirus infections such as fever, cough, shortness of breath, muscle pain, sputum production, diarrhea, sore throat, loss of smell, pneumonia and abdominal pain.

Preferably, the compounds disclosed herein, are used for the treatment and/or prevention of viral infections by *"Severe respiratory syndrome-related coronavirus"* and most preferably for the treatment and/or prevention of viral infections by SARS-CoV-2.

In a particular embodiment of the first, second and third aspects of the invention in the compound for use of formula (I)
- R is a hydroxyl, group,
- R¹, R² and R³ independently represent a hydrogen atom or a phenyl optionally substituted by 1, 2 or 3 groups selected from the group consisting of C₁₋₄ alkyl, C₁₋₄ alkoxy, halogen atoms, acetyl, morpholino and trifluoromethoxy,
- R⁴ is hydrogen, methoxy or phenyl optionally substituted by 1 or 2 groups selected from the group consisting of C₁₋₄ alkyl, halogen atoms, acetyl and trifluoromethoxy, and
- R⁵ is hydrogen, halogen or phenyl optionally substituted by 1, 2 or 3 groups selected from the group consisting of halogen atoms, C₁₋₄ alkyl, C₁₋₄ alkoxy, acetyl, morpholino and trifluoromethoxy.

In a particular embodiment of the first, second and third aspects of the invention in the compound for use of formula (I) at least one of R¹, R², R³, R⁴ and R⁵ is a phenyl group substituted by 1 or 2 groups selected from chlorine atoms, trifluoromethoxy and C₁₋₄ alkyl.

In another embodiment of the first, second and third aspects of the invention in the compound for use of formula (I) R¹ is a hydrogen atom and at least one at least one of R², R³, R⁴ and R⁵ is a phenyl group substituted by 1 group selected C₃₋₄ alkyl.

In still another embodiment of the first, second and third aspects of the invention in the compound for use of formula (I) R¹, R² and R⁵ are hydrogen atoms, one of R³ and R⁴ is a phenyl group substituted by 1 butyl and the other one of R³ and R⁴ is selected from hydrogen atom and methoxy.

In a fourth aspect, the present invention relates to a subgroup of new compounds selected from the group of compounds defined in the first aspect of the invention. Said subgroup is defined by formula (I') 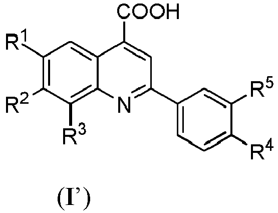 wherein
R¹ is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
R² is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
R³ is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
R⁴ is hydrogen, methoxy or phenyl optionally substituted by 1 or 2 groups selected from the group consisting of C₁₋₄ alkyl, acetyl, halogen atoms and trifluoromethoxy,
R⁵ is hydrogen, halogen or phenyl optionally substituted by 1 group selected from the group consisting of halogen atoms, C₁₋₄ alkyl, acetyl and trifluoromethoxy,
with the proviso that at least one of R¹ and R² is a phenyl group optionally substituted by 1, 2 or 3 groups selected from the group consisting of C₁₋₄ alkyl, C₁₋₄ alkoxy, halogen atoms, acetyl, morpholino and trifluoromethoxy,
and the further proviso that the compound is neither 2-(4-methoxy-phenyl)-quinoline-4-carboxylic acid nor (2,7-diphenyl-quinolin-4-carboxylic acid)
and pharmaceutically acceptable salts thereof.

In one embodiment of the fourth aspect of the invention in the compounds of formula (I') at least one at least one of R¹, R², R³, R⁴ and R⁵ is a phenyl group substituted by 1 or 2 groups selected from chlorine atoms, trifluoromethoxy and C₁₋₄ alkyl.

In another embodiment of the fourth aspect of the invention in the compounds of formula (I') R¹ is a hydrogen atom and at least one at least one of R², R³, R⁴ and R⁵ is a phenyl group substituted by 1 group selected C₃₋₄ alkyl.

In another embodiment of the fourth aspect of the invention in the compounds of formula (I') R¹ and R⁵ are hydrogen atoms, one of R³ and R⁴ is a phenyl group substituted by 1 butyl and the other one of R³ and R⁴ is selected from hydrogen atom and methoxy.

As used herein the term alkyl is used to designate linear or branched hydrocarbon radicals (CₙH₂ₙ₊₁). Examples include methyl, ethyl, n-propyl, i-propyl, n-butyl, sec-butyl, tert-butyl, n-pentyl, 1-methyl-butyl, 2-methyl-butyl, isopentyl, 1-ethylpropyl, 1,1-dimethylpropyl, 1,2-dimethylpropyl, n-hexyl, 1-ethylbutyl, 2-ethylbutyl, 1,1-dimethylbutyl, 1,2-dimethylbutyl, 1,3dimethylbutyl, 2,2-dimethylbutyl, 2,3-dimethylbutyl, 2-methylpentyl and 3-methylpentyl. In a preferred embodiment said alkyl groups have 1 to 6 carbon atoms, most preferably 1 to 4 carbons atoms.

As used herein, the term C₁₋₄ alkoxy is used to designate radicals which contain a linear or branched C₁₋₄ alkyl group linked to an oxygen atom (CₙH₂ₙ₊₁-O-) wherein n ranges from 1 to 4. Preferred alkoxy radicals include methoxy, ethoxy, n-propoxy, i-propoxy, n-butoxy, sec-butoxy and tert-butoxy.

As used herein, the term "allyl" is used to designate the group -CH₂-CH=CH₂.

As used herein, the term "benzyl" refers to the group phenyl-CH₂-.

The term "5-6 membered, monocyclic, heteroaromatic ring comprising 1, 2 or 3 heteroatoms selected from N, O and S" refers to a ring system with only one ring having 5 to 6 members which are selected from carbon atoms, nitrogen, oxygen and sulphur atoms and which is aromatic in nature. Examples of said rings are pyridines, furanes, pyrazines, pyrroles, imidazoles, pyrazoles, oxazoles, thiazoles and thiophenes.

As used herein, the term halogen atom is used to designate an atom selected from the group consisting of chlorine, fluorine, bromine or iodine atom, preferably bromine, fluorine or chlorine atom.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

Further, the term "pharmaceutically acceptable salt" refers to any salt, which, upon administration to the recipient is capable of providing (directly or indirectly) a compound as described herein. For instance, a pharmaceutically acceptable salt of compounds provided herein may be acid addition salts, base addition salts or metallic salts, and they can be synthesized from the parent compound, which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

In another particular embodiment, the viral infection is an infection by *"Severe respiratory syndrome-related coronavirus"* and most preferably by SARS-CoV-2.

The compounds for use according to the invention may be administered by any appropriate route (via), such as, oral (e.g., oral, sublingual, etc.), parenteral (e.g., subcutaneous, intramuscular, intravenous, etc.), vaginal, rectal, nasal, topical, ophtalmic, inhaled, intranasal, intratracheal, pulmonary, etc., preferably oral, inhaled or parenteral, more preferably intravenous.

The compounds for use according to the invention may, in particular be administered by inhalation. In this case, they are advantageously formulated as dry powder compositions and may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred.

In particular, the compounds for use according to the invention are administered as a pharmaceutical composition, which comprises the corresponding (active) compound and one or more pharmaceutically acceptable excipients.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are known by the skilled person.

The pharmaceutically acceptable excipient necessary to manufacture the desired pharmaceutical composition of the invention will depend, among other factors, on the elected administration route. Said pharmaceutical compositions may be manufactured according to conventional methods known by the skilled person in the art.

The compounds for use according to the invention may be administered in a "therapeutically effective amount", i.e. a nontoxic but sufficient amount of the corresponding compound to provide the desired effect. The amount that is "effective" will vary from subject to subject, depending on the age and general condition of the individual, the particular compound administered, and the like. Thus, it is not always possible to specify an exact "therapeutically effective amount". However, an appropriate amount in any individual case may be determined by one of ordinary skill in the art using routine experimentation.

The compounds for use according to the invention will typically be administered once or more times a day, for example 1, 2, 3 or 4 times daily, with typical total daily doses depending on the particular compound and severity of the disease, and may be easily determined by the skilled practitioner.

By way of example, typical total daily doses the compound of the invention are in the range of from 0.1 to 2000 mg/day, preferably from 1 to 600 mg/ day, even more preferably from 1 to 100 mg/day.

The pharmaceutical compositions may be prepared using standard methods such as those described or referred to in the Spanish and US Pharmacopoeias and similar reference texts.

The term "subject" refers to a mammal, e.g., a human.

The compounds for use according to the invention may be administered as the sole active ingredient or in combination with other active ingredients. In a particular embodiment, the compounds are used as the sole active ingredient. In another particular embodiment, the compounds are used in combination with other active ingredients.

In another aspect, the present invention relates to a combination comprising one or more compounds selected useful in the treatment of viral infections by virus of the family *Coronaviridae.* Some compounds that have been disclosed as potentially useful are selected from the group consisting of remdesivir, ribavirin, favipiravir, chloroquine, hydroxychloroquine, lopinavir, ritonavir, umifenovir and, recombinant type I interferon,

The invention also relates to the use of a combination comprising one or more compounds useful in the treatment of viral infections by virus of the family *Coronaviridae.* Some compounds that have been disclosed as potentially useful are selected from the group consisting of remdesivir, ribavirin, favipiravir, chloroquine, hydroxychloroquine, lopinavir, ritonavir, umifenovir and, recombinant type I interferon.

The invention also relates a method of treating and/or preventing viral infections by coronavirus, especially by respiratory syndrome-related coronavirus selected from the species *"Middle East respiratory syndrome-related coronavirus"* (such as MERS-CoV) and *"Severe respiratory syndrome-related coronavirus"* (such as SARS-CoV and SARS-CoV-2) in a subject, comprising administering to said subject a therapeutically effective amount of a combination comprising one or more compounds selected from the group consisting of remdesivir, ribavirin, favipiravir, chloroquine, hydroxychloroquine, lopinavir, ritonavir, umifenovir and, recombinant type I interferon.

The term "combination" refers to a product comprising one or more of the defined compounds, either in a single composition or in several compositions (or units), in which case the corresponding compounds are distributed among the several compositions. Preferably, the combination refers to several compositions, in particular comprising one composition (or unit) per compound (compound as defined above) of the combination. The expression "one or more" when characterizing the combination refers to at least one, preferably 1, 2, 3, 4, or 5 compounds, more preferably, 1, 2 or 3 compounds, even more preferably 1 or 2 compounds.

When the combination is in the form of a single composition, the compounds present in the combination are always administered simultaneously.

When the combination is in the form of several compositions (or units), each of them having at least one of the compounds of the combination, the compositions or (units) may be administered simultaneously, sequentially or separately.

Simultaneous administration means that the compounds or compositions (or units) are administered at the same time.

Sequential administration means that the compounds or compositions (or units) are administered at different time points in a chronologically staggered manner.

Separate administration means that the compounds or compositions (or units) are administered at different time points independently of each other.

In particular, the combinations for use according to the invention are administered as pharmaceutical compositions, which comprise the corresponding (active) compounds and a pharmaceutically acceptable excipient, as previously defined.

The combinations for use according to the invention will typically be administered once or more times a day, for example 1, 2, 3 or 4 times daily, with typical total daily doses depending on the particular compound and severity of the disease, and may be easily determined by the skilled practitioner.

In another particular embodiment, the viral infection is an infection by SARS-CoV-2 virus.

The following examples represent specific embodiments of the present invention. They do not intend to limit in any way the scope of the invention defined in the present description.

### Synthesis of the compounds.

The compounds of general formula (I) of the present invention, can be synthesized as described in detail below:
A mixture of the corresponding isatin (1 eq.), 33% potassium hydroxide (1 mL/mmol) in ethanol (2 mL/mmol) and the 4-substituted acetophenone (1.5 eq.) was heated under reflux for 12-18 h. The reaction mixture was concentrated under reduce pressure and the residue was diluted with water (5 mL) and extracted with ethyl acetate (3 x 15 mL). The aqueous layer was acidified with 1 M hydrochloric acid until pH 3. The precipitated solid was filtered, washed with water and dried to yield the corresponding quinolone-4-carboxylic acid. Then, the obtained quinolone-4-carboxylic acid (0.5 mmol, 1 eq.) was added to a mixture of acetone (2 mL) and potassium carbonate (5 eq.). Next, methyl iodide (5 eq.) was added. The reaction mixture was refluxed with magnetic stirring for 12 h. After this time, it was concentrated under reduced pressure and water was added to the remaining mixture. The product was collected by suction filtration and air-dried as a colored solid.

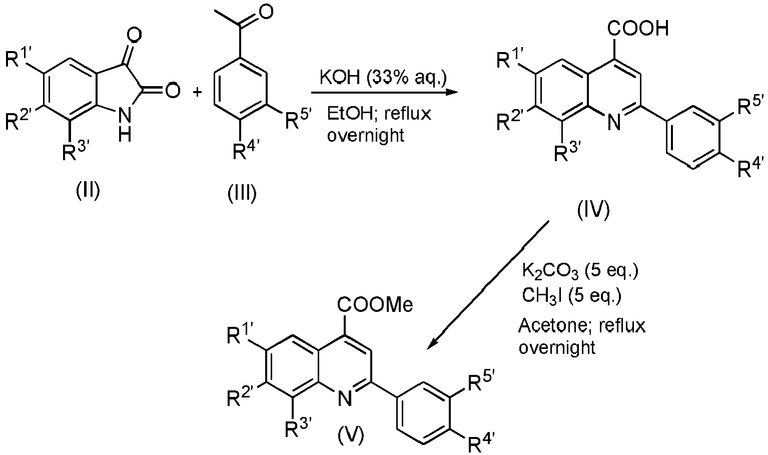

R^{1'} represents either a bromine atom or a group R¹ as defined in claim 1, R^{2'} represents either a bromine atom or a group R² as defined in claim 1, R^{3'} represents either a bromine atom or a group R³ as defined in claim 1, R^{4'} represents either a bromine atom or a group R⁴ as defined in claim 1 and R^{5'} represents either a bromine atom or a group R⁵ as defined in claim 1.

Compounds of formula (III) are either commercially available or may be obtained using a Suzuki reaction between a bromine derivative ((VI) or (VIII)) with a boronic acid ((VII) or (IX)). In formula (VII) (A) represents a phenyl or a 5-6 membered, monocyclic, heteroaromatic ring comprising 1, 2 or 3 heteroatoms selected from N, O and S, which are optionally substituted by 1, 2 or 3 groups selected from the group consisting of halogen atoms, C₁₋₄ alkyl, C₁₋₄ alkoxy, acetyl, morpholino and trifluoromethoxy and in formula (IX) (A) represents a phenyl or a 5-6 membered, monocyclic, heteroaromatic ring comprising 1, 2 or 3 heteroatoms selected from N, O and S, which are optionally substituted by 1 or 2 groups selected from the group consisting of C₁₋₄ alkyl, halogen atoms, acetyl and trifluoromethoxy.

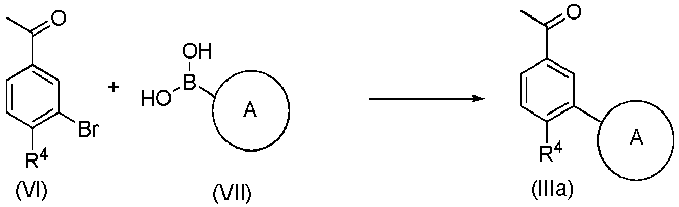

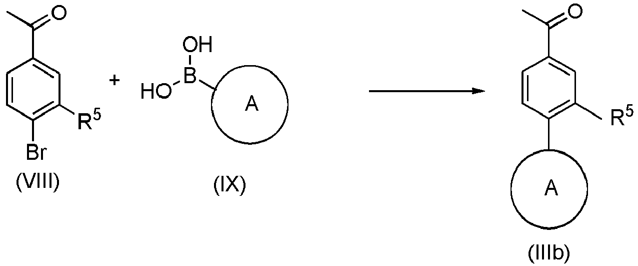

Compounds of formula (II) are either commercially available or may be obtained using a Suzuki reaction between a bromine derivative ((X), (XII) or (XIV)) with a boronic acid ((XI), (XIII) or (XV)). In formulae (XI), (XIII) and (XV) (A) represents a phenyl or a 5-6 membered, monocyclic, heteroaromatic ring comprising 1, 2 or 3 heteroatoms selected from N, O and S, which are optionally substituted by 1, 2 or 3 groups selected from the group consisting of C₁₋₄ alkyl, C₁₋₄ alkoxy, halogen atoms, acetyl, allylamino, morpholino and trifluoromethoxy.

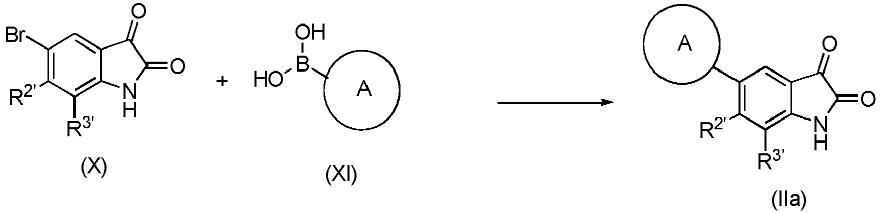

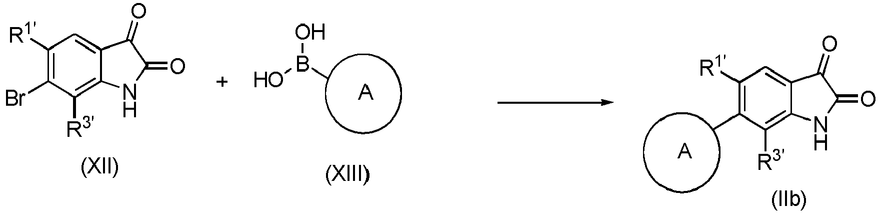

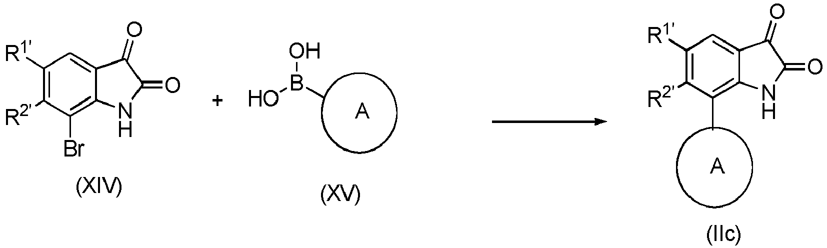

When in the compound of formula (V) any one of R¹, R^{2'}, R^{3'}, R^{4'} and R^{5'} is a bromine atom said bromine atom may be replaced by a desired optionally substituted phenyl group or 5-6 membered, monocyclic, heteroaromatic ring comprising 1, 2 or 3 heteroatoms selected from N, O and S (A) through a Suzuki coupling with the corresponding boronic acid.

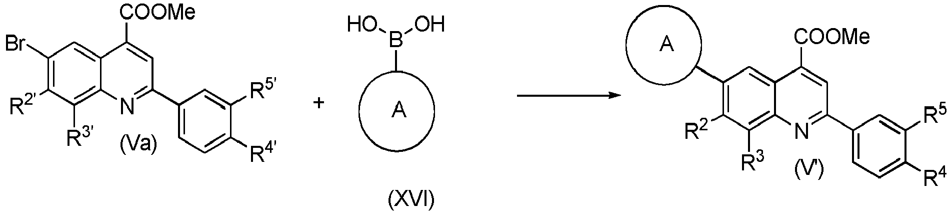

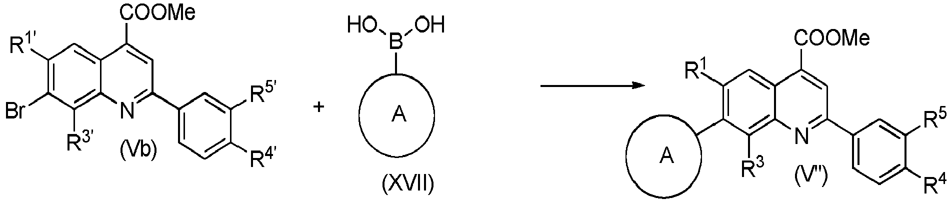

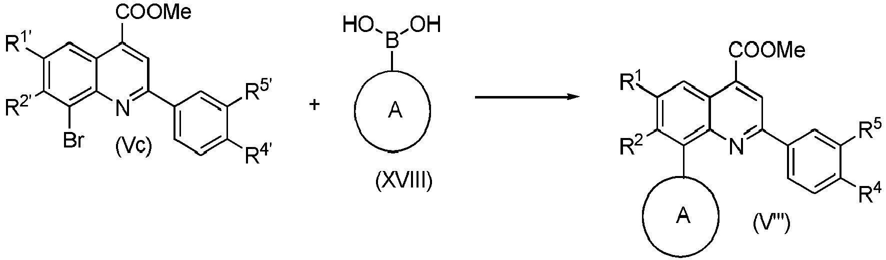

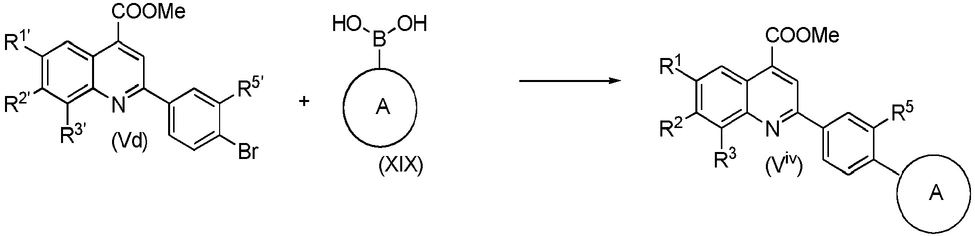

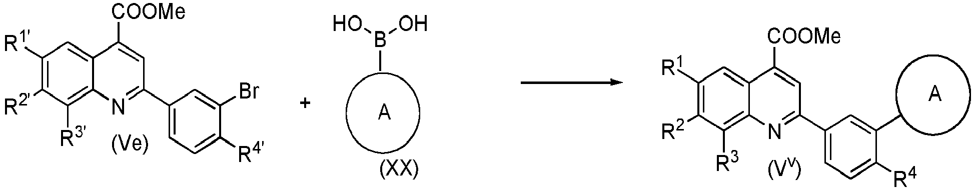

Cross-coupling technology allows the functionalization of an aromatic ring through reactions catalyzed by a transition metal. For example, a Suzuki coupling can be carried out using aryl bromide and a boronic acid coupling partner. Alternatively, couplings between a terminal acetylene and an aryl halide can be carried out by means of the Sonogashira reaction.

### Suzuki coupling conditions

A quinoline halide derivative of formulae (Va), (Vb), (Vc), (Vd) or (Ve) (1 eq), the corresponding boronic acid derivative formulae (XVI), (XVII), (XVIII), (XIX) or (XX) (1.5 eq), K₂CO₃ (6 eq), [Pd(PPh₃)₄] (2 mol%) and 5 mL/mmol of a mixture THF/H2O (4/1) are added in a microwave tube. The reaction mixture is purged with argon and irradiated by MWI for 30 min at 100 °C. The reaction mixture was concentrated under reduced pressure and the residue was diluted with water (5 mL) and extracted with ethyl acetate (3 x 10 mL). The organic layer was dried over MgSO4 anhydrous, filtrated and concentrated. The obtained residue was purified by medium-pressure chromatography (hexane/AcOEt).

### Ester group saponification. General process

The methyl esters of formula (V), (V'), (V"), (V'''), (V^{iv}) and (V^{v}) may be converted into the corresponding carboxylic acids of formula (I) following the saponification reaction explained below:

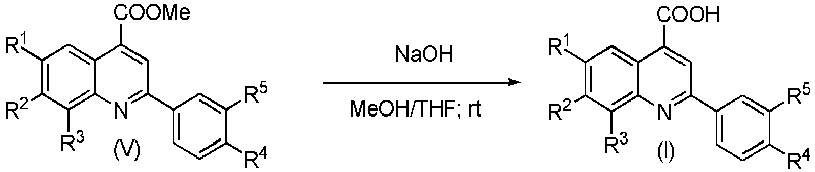

To a solution of the corresponding ester in 13.3 mL/mmol of THF and 6.6 mL/mmol of MeOH is added slowly a solution of NaOH 2M. After 12 h stirring at room temperature, the reaction mixture was concentrated under reduced pressure and the residue was diluted with water (5 mL). The reaction mixture was acidified with 1 M hydrochloric acid until pH 3. The precipitated solid was filtered, washed with water, dried and lyophilized. The compounds was obtained pure without the need of further purification.

### Synthesis of compounds of formula (I) when R is a group selected from allyloxy, benzyloxy and C₁₋₆ alkoxy

NaH (0.94 mmol, 3.0 equiv.) (60 % dispersion in mineral oil) was added portionwise to a solution the corresponding quinoline carboxylic acid (0.31 mmol, 1.0 equiv.) in DMF (5 mL) and then was stirred for 1.5 hour at room temperature. The correspondingbromide (i.e. allyl bromide, benzyl bromide, C₁₋₆ alkyl bromide or mPEG-bromide of formula Br-CH₂-CH₂-(O-CH₂-CH₂-)₍ₙ₋₁₎-OCH₃) (0.94 mmol, 3.0 equiv.) was added dropwise to the previous solution at 0°C and then stirred for 20 hours at room temperature. The reaction was quenched by 50 mL of NH4Cl (sat), extracted by of diethyl ether (3 x 15 mL), washed with brine, dried over MgSO4, and then concentrated to give a crude product which was purified by means of medium-pressure chromatography or by crystallization to give the desired compound.

### Synthesis of compounds of formula (I) when R is a NR'R"

A solution of the corresponding quinoline carboxylic acid (0.75 mmol) in thionyl chloride (1.5 ml) is heated under reflux for 6 h. After this time, the excess thionyl chloride is evaporated to dryness. Then, the residue is dissolved in anhydrous THF (2 ml), and the corresponding amine NHR'R" (0.5 mmol) and propylene oxide (7.5 mmol) are added to the solution. The reaction is stirred at room temperature overnight. Lastly, the excess solvent is removed under vacuum and the solid formed is washed with water. The synthesized product is purified by successive washes with the suitable solvent or by means of medium-pressure chromatography.

### Examples

HPLC-MS chromatographic data mentioned in the following examples were obtained with a Waters 2695 kit coupled to a Waters Micromass ZQ spectrometer. The separation was performed with a Sunfire C18 column (3.5 µm, 4.6 mm x 50 mm), a flow of 1.0 mL/min and as a mobile phase, a gradient from A (CH₃CN) to B (H₂O) with 0.1% acid formic as an additive. Mass spectra were obtained using the ESI electrospray technique. The peaks corresponding to the molecular ion have been indicated as ([M]⁺), while those corresponding to the molecular ion plus one unit as ([M⁺H]⁺).

### Example 1: 2-(4-methoxyphenyl)-6-phenylquinoline-4-carboxylic acid

The product was prepared according to the methods described above and obtained as a yellow solid with a yield of 43%.
m.p.: 272.8 °C
¹H NMR (300 MHz, DMSO-d₆) δ 8.98 (d, *J* = 2.0 Hz, 1H, H₅), 8.46 (s, 1H, H₃), 8.29 (m, 2H, H₇H₈), 8.17 (m, 2H, H_{2'}H_{6'}), 7.80 (m, 2H, H_{2"}H_{6"}), 7.55 (t, *J* = 7.5 Hz, 2H, H_{3"}H_{5"}), 7.44 (t, *J* = 7.5 Hz, 1H, H_{4"}), 7.12 (m, 2H, H_{3'}H_{5'}), 3.86 (s, 3H, H_{7'}).
¹³C NMR (75 MHz, DMSO-d₆) δ 167.6 (-COOH), 160.9 (C_{4'}), 155.4 (C₂), 147.8 (C₄), 139.6 (C_{1"}), 138.7 (C₆), 137.3 (C₈ₐ₎, 130.2 (C₈), 130.2 (C_{1'}), 129.2 (C_{2'}C_{6'}), 129.0 (C_{4"}), 128.6 (C_{3"}C_{5"}), 127.9 (C₄ₐ), 127.1 (C_{2"}C_{6"}), 123.4 (C₃), 122.8 (C₅), 119.3 (C₇), 114.3 (C_{3'}C_{5'}), 55.3 (C_{7'}).
HPLC (Sunfire C18, 60-95% of acetonitrile in water, 10 min): t_{R} = 3.31 min.
LC-MS (m/z): 356.1 ([M⁺H]⁺).

### Example 2: 6-(4-ethylphenyl)-2-(4-methoxyphenyl)quinoline-4-carboxylic acid

The product was prepared according to the methods described above and obtained as a yellow solid with a yield of 95%.
m.p.: 254.9 °C
¹H NMR (300 MHz, DMSO-d₆) δ 8.88 (d, *J* = 1.9 Hz, 1H, H₅), 8.43 (s, 1H, H₃), 8.28 (m, 2H, H_{2"}H_{6"}), 8.16 (d, *J* = 8.8 Hz, 1H, H₈), 8.11 (dd, *J* = 8.8, 2.0 Hz, 1H, H₇), 7.71 (m, 2H, H_{2'}H_{6'}), 7.38 (m, 2H, H_{3"}H_{5"}), 7.12 (m, 2H, H_{3'}H_{5'}), 3.86 (s, 3H, H₇), 2.68 (q, *J* = 7.6 Hz, 2H, H_{7"}), 1.23 (t, J = 7.6 Hz, 3H, H_{8"}).
¹³C NMR (75 MHz, DMSO-d₆) δ 168.1 (COO-), 161.2 (C_{4'}), 155.6 (C₂), 148.1 (C₄), 144.0 (C₈ₐ₎, 138.9 (C_{4"}), 137.3 (C₆), 130.7 (C_{1"}), 130.4 (C_{1'}), 129.4 (C₈), 128.9 (C₅), 128.9 (C_{2"}C_{6"}), 128.9 (C_{2'}C_{6'}), 127.3 (C_{3"}C_{5"}), 123.8 (C₇), 122.9 (C₄ₐ), 119.4 (C₃), 114.7 (C_{3'}C_{5'}), 55.7 (C_{7'}), 28.2 (C_{7"}), 15.91 (C_{8"}).
HPLC (Sunfire C18, 60-95% of acetonitrile in water, 10 min): t_{R} = 4.06 min.
LC-MS (m/z): 384.2 ([M⁺H]⁺).

### Example 3:6-(4-butylphenyl)-2-(4-methoxyphenyl)quinoline-4-carboxylic acid.

The product was prepared according to the methods described above and obtained as a yellow solid with a yield of 60%.
m.p.: 124.8 °C
¹H NMR (300 MHz, DMSO-d₆) δ 8.86 (d, *J* = 2.1 Hz, 1H, H₅), 8.46 (s, 1H, H₃), 8.28 (m, 2H, H_{2"}H_{6"}), 8.14 (m, 2H, H₇H₈), 7.70 (m, 2H, H_{2'}H_{6'}), 7.35 (d, *J* = 8.0 Hz, 2H, H_{3"}H_{5"}), 7.13 (m, 2H, H_{3'}H_{5'}), 3.86 (s, 3H, H_{7'}), 2.64 (t, *J* = 7.6 Hz, 2H, H_{7"}), 1.59 (m, 2H, H_{8"}), 1.32 (m, 2H, H_{9"}), 0.91 (t, *J* = 7.4 Hz, 3H, H_{10"}).
¹³C NMR (75 MHz, DMSO-d₆) δ 167.6 (COO-), 160.9 (C_{4'}), 155.2 (C₂), 147.6 (C_{4"}), 142.3 (C₄), 138.7 (C₈ₐ₎, 137.2 (C₆), 136.9 (C_{1"}), 130.1 (C_{1'}), 129.3 (C₈), 129.1 (C_{2"}C_{6"}), 129.0 (C₅), 128.7 (C_{2'}C_{6'}), 126.9 (C_{3"}C_{5"}), 123.5 (C₄ₐ), 122.4 (C₇), 119.3 (C₃), 114.4 (C_{3'}C_{5'}), 55.3 (C_{7'}), 34.3 (C_{7"}), 33.0 (C_{8"}), 21.8 (C_{9"}), 13.8 (C_{10"}).
HPLC (Sunfire C18, 60-95% of acetonitrile in water, 10 min): t_{R} = 9.15 min.
LC-MS (m/z): 412.2 ([M⁺H]⁺).

### Example 4 : 6-(4-(tert-butyl)phenyl)-2-(4-methoxyphenyl)quinoline-4-carboxylic acid

The product was prepared according to the methods described above and obtained as a orange solid with a yield of 78%.
m.p.: 164.0 °C
¹H NMR (300 MHz, DMSO-d₆) δ 8.88 (d, *J* = 2.0 Hz, 1H, H₅), 8.46 (s, 1H, H₃), 8.28 (m, 2H, H_{2'}H_{6'}), 8.18 (d, *J* = 8.8 Hz, 1H, H₈), 8.11 (dd, *J* = 8.8, 2.0 Hz, 1H, H₇), 7.71 (m, 2H, H_{2"}H_{6"}), 7.55 (m, 2H, H_{3"}H_{5"}), 7.12 (m, 2H, H_{3'}H_{5'}), 3.86 (s, 3H, H₇), 1.33 (s, 9H, H_{(tBu)}).
¹³C NMR (75 MHz, DMSO-d₆) δ 167.6 (COO-), 160.9 (C_{4'}), 155.2 (C₂), 150.5 (C_{4"}), 147.6 (C₄), 138.7 (C₈ₐ₎, 137.2 (C₆), 136.7 (C_{1"}), 130.1 (C_{1'}), 130.0 (C₈), 129.2 (C₅), 128.7 (C_{2"}C_{6"}), 126.8 (C_{2'}C_{6'}), 126.0 (C_{3"}C_{5"}), 123.4 (C₄ₐ), 122.5 (C₇), 119.3 (C₃), 114.4 (C_{3'}C_{5'}), 55.3 (C_{7'}), 34.3 (C_{7"}), 31.1 (C_{tBu}).
HPLC (Sunfire C18, 60-95% of acetonitrile in water, 10 min): t_{R} = 8.20 min.
LC-MS (m/z): 412.2 ([M]⁺)

### Example 5: 6-(4-acetylphenyl)-2-(4-methoxyphenyl)quinoline-4-carboxylic acid

The product was prepared according to the methods described above and obtained as an orange solid with a yield of 87%.
m.p.: 246.9 °C
¹H NMR (300 MHz, DMSO-d₆) δ 13.9 (s, 1H, -COOH), 8.98 (d, *J* = 1.7 Hz, 1H, H₅), 8.48 (s, 1H, H₃), 8.32 (m, 2H, H_{2'}H_{6'}), 8.25 (m, 2H, H_{3"}H_{5"}), 8.12 (m, 2H, H_{2"}H_{6"}), 7.98 (m, 2H, H_{2'}H_{6'}), 7.15 (m, 2H, H_{3'}H_{5'}), 3.86 (s, 3H, H_{7'}), 2.64 (s, 3H, H_{8"}).
¹³C NMR (75 MHz, DMSO-d₆) δ 197.5 (C_{7"}), 167.5 (COO-), 161.0 (C_{4'}), 155.8 (C₂), 148.1 (C₄), 143.9 (C₈ₐ₎, 137.4 (C₆), 137.3 (C_{1"}), 135.9 (C_{4"}), 130.5 (C₈), 130.3 (C_{1'}), 130.1 (C₅), 129.1 (C_{2"}C_{6"}), 128.7 (C_{2'}C_{6'}), 127.2 (C_{3"}C_{5"}), 123.5 (C₇), 124.3 (C₄ₐ), 119.5 (C₃), 114.4 (C_{3'}C_{5'}), 55.3 (C_{7'}), 26.8 (C_{8"}).
HPLC (Sunfire C18, 60-95% of acetonitrile in water, 10 min): t_{R} = 1.70 min.
LC-MS (m/z): 398.2 ([M⁺H]⁺).

### Example 6: 6-(3,4-dichlorophenyl)-2-(4-methoxyphenyl)quinoline-4-carboxylic acid

The product was prepared according to the methods described above and obtained as a yellow solid with a yield of 83%.
m.p: 281.5 °C
¹H NMR (300 MHz, DMSO-d₆) δ 8.91 (d, *J* = 1.7 Hz, 1H, H₅), 8.47 (s, 1H, H₃), 8.34 (m, 2H, H₇H₈), 8.24 (m, 2H, H_{2'}H_{6'}), 8.06 (s, 1H, H_{2"}), 7.80 (d, *J* = 1.2 Hz, 2H, H_{5"}H_{6"}), 7.17 (m, 2H, H_{3'}H_{5'}), 3.86 (s, 3H, H_{7'}).
¹³C NMR (75 MHz, DMSO-d₆) δ 167.4 (-COOH), 161.0 (C_{4'}), 155.8 (C₂), 148.1 (C₄), 140.2 (C₈ₐ₎, 137.6 (C₆), 136.0 (C_{1"}), 131.9 (C_{1'}), 131.2 (C_{3"}), 130.7 (C₈), 130.7 (C₅), 130.1 (C₇), 129.0 (C_{4"}), 128.8 (C_{5"}), 128.7 (C_{2'}C_{6'}), 127.3 (C_{2"}), 123.5 (C₄ₐ), 123.6 (C_{6"}), 119.4 (C₃), 114.4 (C_{3'}C_{5'}), 55.3 (C_{7'}).
HPLC (Sunfire C18, 60-95% of acetonitrile in water, 10 min): t_{R} = 5.33 min.
LC-MS (m/z): 424.2 ([M]⁺).

### Example 7: 2-(4-methoxyphenyl)-6-(3-(trifluoromethoxy)phenyl)quinoline-4-carboxylic acid

The product was prepared according to the methods described above and obtained as a yellow solid with a yield of 89%.
m.p.: 169.3 °C
¹H NMR (300 MHz, DMSO-d₆) δ 8.92 (d, *J* = 1.9 Hz, 1H, H₅), 8.47 (s, 1H, H₃), 8.28 (m, 2H, H₇H₈), 8.18 (m, 2H, H_{2'}H_{6'}), 7.83 (m, 1H, H_{6"}), 7.75 (s, 1H, H_{2"}), 7.68 (t, *J* = 8.0 Hz, 1H, H_{5"}), 7.45 (m, 1H, H_{4"}), 7.12 (m, 2H, H_{3'}H_{5'}), 3.86 (s, 3H, H_{7'}).
¹³C NMR (75 MHz, DMSO-d₆) δ 167.5 (COO-), 161.0 (C_{4'}), 155.8 (C₂), 149.0 (C_{3"}), 148.1 (C₄), 142.0 (C₈ₐ₎, 137.3 (C_{1"}), 137.0 (C₆), 131.2 (C_{1'}), 130.3 (C₈), 130.1 (C₅), 129.1 (C_{5"}), 128.7 (C_{2'}C_{6'}), 126.2 (C₇), 123.5 (C₄ₐ), 123.3 (C_{6"}), 120.3 (C_{7"}), 119.6 (C_{4"}), 119.6 (C_{2"}), 119.5 (C₃), 114.4 (C_{3'}C_{5'}), 55.3 (C_{7'}).
HPLC (Sunfire C18, 60-95% of acetonitrile in water, 10 min): t_{R} = 4.53 min.
LC-MS (m/z): 440.2 ([M⁺H]⁺).

### Example 8: 2-(4-methoxyphenyl)-6-(4-propoxyphenyl)quinoline-4-carboxylic acid

The product was prepared according to the methods described above and obtained as a red solid with a yield of 85%.
m.p.: 205.3 °C
¹H NMR (300 MHz, DMSO-d₆) δ 9.04 (d, *J* = 2.0 Hz, 1H, H₅), 8.40 (s, 1H, H₃), 8.25 (d, *J* = 8.8 Hz, 1H, H₈), 8.22 - 8.15 (m, 2H, H_{3"}H_{5"}), 8.11 - 8.04 (m, 2H, H_{2"}H_{6"}), 8.01 (dd, *J* = 8.8, 2.1 Hz, 1H, H₇), 7.85 (dq, *J* = 8.6, 2.0 Hz, 2H, H_{2'}H_{6'}), 7.11 - 7.02 (m, 2H, H_{3'}H_{5'},), 4.08 (s, 3H, H₉), 3.89 (s, 3H, H_{7'}), 2.66 (s, 3H, H_{8"}).
¹³C NMR (75 MHz, DMSO-d₆) δ 167.8 (COO-), 160.8 (C_{4'}), 158.7 (C_{4"}), 155.0 (C₂), 147.5 (C₄), 138.2 (C₈ₐ₎, 138.0 (C₆), 131.7 (C_{1"}), 130.4 (C_{1'}), 130.0 (C₈), 128.9 (C₅), 128.6 (C_{2'}C_{6'}), 128.1 (C_{2"}C_{6"}), 123.6 (C₄ₐ), 121.9 (C₇), 118.9 (C₃), 115.1 (C_{3"}C_{5"}), 114.3 (C_{3'}C_{5'}), 69.0 (C_{7"}), 55.3 (C_{7'}), 22.0 (C_{8"}), 10.4 (C_{9"}).
HPLC (Sunfire C18, 60-95% of acetonitrile in water, 10 min): t_{R} = 4.15 min.
LC-MS (m/z): 414.4 ([M⁺H]⁺).

### Example 9: 2-(4-methoxyphenyl)-6-(3,4,5-trimethoxyphenyl)quinoline-4-carboxylic acid

The product was prepared according to the methods described above and obtained as a yellow solid with a yield of 97%.
m.p.: 235.6 °C
¹H NMR (300 MHz, DMSO-d₆) δ 8.85 (d, *J* = 1.3 Hz, 1H, H₅), 8.45 (s, 1H, H₃), 8.29 (m, 2H, H_{2'}H_{6'}), 8.17 (m, 2H, H₇H₈), 7.13 (m, 2H, H_{3'}H_{5'}), 7.04 (s, 2H, H_{2"}H_{6"}), 3.90 (s, 6H, H_{7"}H_{9"}), 3.86 (s, 3H, H_{8"}), 3.73 (s, 3H, H_{7'}).
¹³C NMR (75 MHz, DMSO-d₆) δ 167.7 (COO-), 160.9 (C_{4'}), 155.3 (C₂), 153.4 (C_{3"}C_{5"}), 147.8 (C₄), 139.0 (C₈ₐ₎, 137.7 (C₆), 137.4 (C_{4"}), 135.5 (C_{1"}), 130.3 (C_{1'}), 129.8 (C₅), 129.6 (C₈), 128.7 (C_{2'}C_{6'}), 123.3 (C₄ₐ), 122.8 (C₇), 119.2 (C₃), 114.4 (C_{3'}C_{5'}), 104.8 (C_{2"}C_{6"}), 60.1 (C_{8"}), 56.1 (C_{7"}C_{9"}), 55.4 (C_{7'}).
HPLC (Sunfire C18, 60-95% of acetonitrile in water, 10 min): t_{R} = 1.67 min.
LC-MS (m/z): 446.3 ([M⁺H]⁺).

### Example 10: 2-(4-methoxyphenyl)-6-(4-morpholinophenyl)quinoline-4-carboxylic acid.

The product was prepared according to the methods described above and obtained as a black solid with a yield of 87%.
m.p: n.d.
¹H NMR (300 MHz, DMSO-d₆) δ 8.92 (d, *J* = 1.9 Hz, 1H, H₅), 8.21 (m, 2H, H_{2'}H_{6'}), 8.12 (s, 1H, H₃), 8.01 (m, 2H, H₈H₇), 7.60 (m, 4H, H_{3"}H_{5"}H_{2"}H_{6"}), 7.09 (m, 2H, H_{3'}H_{5'}), 3.84 (s, 3H, H_{7'}), 3.76 (t, *J* = 4.7 Hz, 4H, H_{8"}H_{9"}), 3.17 (t, *J* = 4.7 Hz, 4H, H_{7"}H_{10"}).
¹³C NMR (75 MHz, DMSO-d₆) δ 169.6 (COO-), 160.7 (C_{4'}), 155.1 (C₂), 150.8 (C_{4"}), 147.7 (C₄), 137.1 (C₈ₐ₎, 131.9 (C₆), 131.7 (C_{1"}), 130.6 (C_{1'}), 129.1 (C₈), 129.0 (C₅), 128.7 (C_{2"}C_{6"}), 128.0 (C₇), 127.7 (C_{2'}C_{6'}), 123.5 (C₄ₐ), 117.0 (C₃), 115.7 (C_{3"}C_{5"}), 114.5 (C_{3'}C_{5'}), 66.4 (C_{8"}C_{9"}), 55.6 (C_{7'}), 48.4 (C_{7"}C_{10"}).
HPLC (Sunfire C18, 60-95% of acetonitrile in water, 10 min): t_{R} = 1.59 min.
LC-MS (m/z): 441.5 ([M+H]⁺)

### Example 11: 2,6-diphenylquinoline-4-carboxylic acid

The product was prepared according to the methods described above and obtained as a yellow solid with a yield of 91%.
m.p.: 208.4 °C
¹H NMR (300 MHz, DMSO-d6) δ 8.94 (d*, J* = 1.5 Hz, 1H, H₅), 8.51 (s, 1H, H₃), 8.31 (m, 2H, H_{2'}H_{6'}), 8.25 (d, *J* = 8.8 Hz, 1H, H₈), 8.17 (dd, *J* = 8.8, 2.0 Hz, 1H, H₇), 7.81 (m, 2H, H_{3"}H_{5"}), 7.57 (m, 5H, H_{4"}H_{2"}H_{6"}H_{3"}H_{5"}), 7.46 (m, 1H, H_{4'}).
¹³C NMR (75 MHz, DMSO-d₆) δ 167.6 (-COO), 155.8 (C₂), 147.9 (C₄), 139.5 (C₈ₐ₎, 139.3 (C_{1"}), 137.8 (C₆), 137.4 (C_{1'}), 130.4 (C_{4"}), 130.1 (C₇), 129.5 (C₅), 129.2 (C_{3'}C_{5'}), 129.0 (C_{3"}C_{5"}), 128.1 (C_{4'}), 127.3 (C_{2"}C_{6"}), 127.2 (C_{2'}C_{6'}), 123.8 (C₈), 122.9 (C₄ₐ), 119.8 (C₃). HPLC (Sunfire C18, 60-95% of acetonitrile in water, 10 min): t_{R} = 2.84 min.
LC-MS (m/z): 326.3 ([M⁺H]⁺).

### Example 12: 6-(4-ethylphenyl)-2-phenylquinoline-4-carboxylic acid

The product was prepared according to the methods described above and obtained as a yellow solid with a yield of 98%.
m.p.: 228.7 °C
¹H NMR (300 MHz, DMSO-d₆δ 8.91 (d, *J* = 1.9 Hz, 1H, H₅), 8.50 (s, 1H, H₃), 8.30 (m, 2H, H_{2'}H_{6'}), 8.21 (d, *J* = 8.8 Hz, 1H, H₈), 8.13 (dd, *J* = 8.8, 1.9 Hz, 1H, H₇), 7.70 (m, 2H, H_{3"}H_{5"}), 7.56 (m, 3H, H_{4'}H_{2"}H_{6"}), 7.37 (d, *J* = 8.0 Hz, 2H, H_{3'}H_{5'}), 2.67 (q, *J* = 7.6 Hz, 2H, H_{7"}), 1.22 (t, *J* = 7.6 Hz, 3H, H_{8"}).
¹³C NMR (75 MHz, DMSO-d₆) δ 167.5 (-COO), 155.5 (C₂), 147.8 (C₄), 143.8 (C₈ₐ₎, 139.2 (C₆), 137.8 (C_{1"}), 137.2 (C_{4"}), 136.9 (C_{1'}), 130.3 (C₈), 129.9 (C₇), 129.3 (C_{4'}), 129.0 (C_{2"}C_{6"}), 128.6 (C_{3'}C_{5'}), 127.1 (C_{3"}C_{5"}), 127.0 (C_{2'}C_{6'}), 123.8 (C₄ₐ), 122.4 (C₅), 119.7 (C₃), 27.8 (C_{7"}), 15.5 (C_{8"}).
HPLC (Sunfire C18, 60-95% of acetonitrile in water, 10 min): t_{R} = 5.28 min.
LC-MS (m/z): 354.2 ([M⁺H]⁺).

### Example 13: 6-(4-butylphenyl)-2-phenylquinoline-4-carboxylic acid

The product was prepared according to the methods described above and obtained as a yellow solid with a yield of 77%.
m.p.: 213.6 °C
¹H NMR (300 MHz, DMSO-d₆) δ 13.99 (s, 1H, -COOH), 8.92 (d, *J* = 2.0 Hz, 1H, H₅), 8.51 (s, 1H, H₃), 8.31 (dd, *J* = 8.1, 1.5 Hz, 2H, H_{2'}H_{6'}), 8.23 (d, *J* = 8.8 Hz, 1H, H₈), 8.16 (dd, *J* = 8.8, 2.0 Hz, 1H, H₇), 7.72 (d, *J* = 8.2 Hz, 2H, H_{3"}H_{5"}), 7.57 (m, 3H, H_{4'}H_{2"}H_{6"}), 7.37 (d, *J* = 8.2 Hz, 2H, H_{3'}H_{5'}), 2.66 (t, *J* = 7.6 Hz, 2H, H_{7"}), 1.61 (p, *J* = 7.6 Hz, 2H, H_{8"}), 1.35 (sext, *J* = 7.6 Hz, 2H, H_{9"}), 0.92 (t, *J* = 7.7 Hz, 3H, H_{10"}).
¹³C NMR (75 MHz, DMSO-d₆) δ 167.6 (-COO), 155.6 (C₂), 147.8 (C₄), 142.5 (C₈ₐ₎, 139.2 (C₆), 137.9 (C_{1"}), 137.2 (C_{4"}), 136.9 (C_{1'}), 130.4 (C₈), 130.0 (C₇), 129.3 (C_{4'}), 129.2 (C_{2"}C_{6"}), 129.0 (C_{3'}C_{5'}), 127.2 (C_{3"}C_{5"}), 127.0 (C_{2'}C_{6'}), 123.9 (C₄ₐ), 124.5 (C₅), 119.7 (C₃), 34.5 (C_{7"}), 33.0 (C_{8"}), 21.8 (C_{9"}), 13.8 (C_{10"}).
HPLC (Sunfire C18, 60-95% of acetonitrile in water, 10 min): t_{R} = 7.92 min.
LC-MS (m/z): 382.5 ([M+H]⁺)

### Example 14: 6-(4-(tert-butyl)phenyl)-2-phenylquinoline-4-carboxylic acid

The product was prepared according to the methods described above and obtained as a yellow solid with a yield of 94%.
m.p.: 274.2 °C
¹H NMR (300 MHz, DMSO-d₆) δ 8.93 (d, *J* = 2.0 Hz, 1H, H₅), 8.50 (s, 1H, H₃), 8.32 (m, 2H, H_{2'}H_{6'}), 8.24 (d, *J* = 8.9 Hz, 1H, H₈), 8.17 (dd, *J* = 8.9, 2.0 Hz, 1H, H₇), 7.75 (m, 2H, H_{3"}H_{5"}), 7.57 (m, 5H, H_{4'}H_{3'}H_{5'}H_{2"}H_{6"}), 1.36 (s, 9H, H_{*t*Bu}).
¹³C NMR (75 MHz, DMSO-d₆) δ 167.5 (-COO), 155.5 (C₂), 150.6 (C₄), 147.8 (C₈ₐ₎, 139.1 (C₆), 137.8 (C_{1"}), 136.6 (C_{4"}), 130.3 (C_{1'}), 129.9 (C₈), 129.3 (C₇), 129.0 (C_{2"}C_{6"}), 127.1 (C_{3'}C_{5'}), 126.8 (C_{3"}C_{5"}), 126.0 (C_{2'}C_{6'}), 126.0 (C_{4'}), 123.8 (C₄ₐ), 122.5 (C₅), 119.7 (C₃), 34.2 (C_{7"}), 31.1 (C_{*t*Bu}).
HPLC (Sunfire C18, 60-95% of acetonitrile in water, 10 min): t_{R} = 7.04 min.
LC-MS (m/z): 382.2 ([M⁺H]⁺).

### Example 15: 6-(4-acetylphenyl)-2-phenylquinoline-4-carboxylic acid

The product was prepared according to the methods described above and obtained as a yellow solid with a yield of 74%.
m.p.: 253.3 °C
¹H NMR (300 MHz, DMSO-d₆) δ 14.1 (s, 1H, -COOH) 9.08 (d, *J* = 1.5 Hz, 1H, H₅), 8.54 (s, 1H, H₃), 8.33 (m, 2H, H_{2'}H_{6'}), 8.24 (m, 2H, H₇H₈), 8.13 (d, *J* = 8.5 Hz, 2H, H_{3"}H_{5"}), 7.96 (d, *J* = 8.5 Hz, 2H, H_{2"}H_{6"}), 7.59 (m, 3H, H_{4'}H_{3'}H_{5'}), 2.66 (s, 3H, H_{8"}).
¹³C NMR (75 MHz, DMSO-d₆) δ 197.9 (C_{7"}), 167.7 (-COO), 156.5 (C₂), 149.0 (C₄), 144.1 (C₈ₐ), 138.2 (C₆), 138.0 (C_{1"}), 137.8 (C_{4"}), 136.3 (C_{1'}), 130.8 (C₈), 130.4 (C₇), 129.5 (C_{4'}), 129.4 (C_{2"}C_{6"}), 129.3 (C_{3'}C_{5'}), 127.6 (C_{3"}C_{5"}), 127.5 (C_{2'}C_{6'}), 124.0 (C₄ₐ), 123.9 (C₅), 120.2 (C₃), 27.1 (C_{8"}).
HPLC (Sunfire C18, 60-95% of acetonitrile in water, 10 min): t_{R} = 2.02 min.
LC-MS (m/z): 368.3 ([M⁺H]⁺).

### Example 16: 6-(3,4-dichlorophenyl)-2-phenylquinoline-4-carboxylic acid

The product was prepared according to the methods described above and obtained as a yellow solid with a yield of 84%.
m.p.: 183.4 °C
¹H NMR (300 MHz, DMSO-d₆) δ 8.98 (d, *J* = 1.6Hz, 1H, H₅), 8.35 (s, 1H, H₃), 8.28 (m, 2H, H_{2'}H_{6'}), 8.17 (d, *J* = 8.9 Hz, 1H, H₈), 8.12 (dd, *J* = 8.9, 1.9 Hz, 1H, H₇), 8.03 (m, 1H, H_{2"}), 7.77 (m, 1H, H_{5"}), 7.55 (m, 4H, H_{4'}H_{3'}H_{5'}H_{6"}).
¹³C NMR (75 MHz, DMSO-d₆) δ 168.2 (-COO), 156.2 (C₂), 148.0 (C₄), 140.4 (C₈ₐ), 138.2 (C₆), 135.6 (C_{1"}), 131.9 (C_{1'}), 131.2 (C_{4"}), 131.2 (C_{5"}), 130.6 (C₇), 130.3 (C_{3"}), 129.9 (C₅), 129.0 (C_{3'}C_{5'}), 128.8 (C_{2"}), 128.6 (C_{4'}), 127.3 (C₈), 127.2 (C_{2'}C_{6'}), 124.3 (C_{6"}), 124.2 (C₄ₐ), 118.7 (C₃).
HPLC (Sunfire C18, 60-95% of acetonitrile in water, 10 min): t_{R} = 5.88 min.
LC-MS (m/z): 394.2 ([M+H]⁺).

### Example 17: 2-phenyl-6-(3-(trifluoromethoxy)phenyl)quinoline-4-carboxylic acid

The product was prepared according to the methods described above and obtained as a yellow solid with a yield of 88%.
m.p.: 164.3 °C
¹H NMR (300 MHz, DMSO-d₆) δ 9.04 (d, *J =* 2.1 Hz, 1H, H₅), 8.30 (s, 1H, H₃), 8.27 (m, 2H, H_{2'}H_{6'}), 8.17 (d, *J =* 8.8 Hz, 1H, H₈), 8.11 (dd, *J* = 8.8, 2.1 Hz, 1H, H₇), 7.84 (m, 2H, H_{6"}), 7.73 (m, 2H, H_{2"}), 7.68 (t, *J =* 8.0 Hz, 1H, H_{5"}), 7.54 (m, 3H, H_{3'}H_{5'}H_{4'}), 7.43 (m, 2H, H_{4"}).
¹³C NMR(75 MHz, DMSO-d₆) δ 168.8 (-COO), 156.1 (C₂), 149.0 (C_{3"}), 148.0 (C₄), 144.1 (C₈ₐ), 142.2 (C₆), 138.4 (C_{1"}), 136.2 (C_{1'}), 131.1 (C₈), 130.2 (C₇), 129.7 (C_{4'}), 128.9 (C_{3'}C_{5'}), 128.5 (C_{5"}), 127.1 (C_{2'}C_{6'}), 126.2 (C₅), 124.6 (C₄ₐ), 124.4 (C_{6"}), 120.4 (C_{7"}), 120.1 (C₃), 119.4 (C_{2"}), 118.4 (C_{4"}).
HPLC (Sunfire C18, 60-95% of acetonitrile in water, 10 min): t_{R} = 5.33 min.
LC-MS (m/z): 410.3 ([M+H]⁺).

### Example 18: 2-([1,1'-biphenyl]-4-yl)quinoline-4-carboxylic acid

The product was prepared according to the methods described above and obtained as a yellow solid with a yield of 83%.
m.p.: 291.3 °C
¹H NMR (300 MHz, DMSO-d₆) δ 14.0 (s, 1H, -COOH), 8.67 (dt, *J =* 8.5, 1.9 Hz, 1H, H₅), 8.53 (d, *J =* 2.2 Hz, 1H, H₃), 8.41 (m, 2H, H_{2'}H_{6'}), 8.19 (dt, *J* = 8.6, 1.8 Hz, 1H, H₈), 7.88 (m, 3H, H₆H_{3'}H_{5'}), 7.78 (m, 2H, H_{2"}H_{6"}), 7.72 (ddt, *J =* 8.8, 6.8, 1.7 Hz, 1H, H₇), 7.52 (m, 2H, H_{3"}H_{5"}), 7.42 (m, 1H, H_{4"}).
¹³C NMR (75 MHz, DMSO-d₆) δ 167.6 (COO-), 155.3 (C₂), 148.3 (C₈ₐ), 141.5 (C₄), 139.3 (C_{4'}), 137.6 (C_{1"}), 136.8 (C_{1'}), 130.2 (C_{4"}), 129.7 (C₇), 129.0 (C_{2"}C_{6"}), 127.9 (C₈), 127.7 (C_{3"}C_{5"}), 127.7 (C₆), 127.2 (C_{3'}C_{5'}), 126.7 (C_{2'}C_{6'}), 125.4 (C₅), 123.4 (C₄ₐ), 119.0 (C₃).
HPLC (Sunfire C18, 60-95% of acetonitrile in water, 10 min): t_{R} = 3.81 min.
LC-MS (m/z): 326.2 ([M+H]⁺).

### Example 19: 2-(4'-ethyl-[1,1'-biphenyl]-4-yl)quinoline-4-carboxylic acid

The product was prepared according to the methods described above and obtained as a yellow solid with a yield of 87%.
m.p.: 251.6 °C
¹H NMR (300 MHz, DMSO-d₆) δ 8.66 (dd, *J =* 8.5, 1.4 Hz, 1H, H₅), 8.50 (s, 1H, H₃), 8.39 (m, 2H, H_{2'}H_{6'}), 8.18 (dd, *J* = 8.7, 1.3 Hz, 1H, H₈), 7.87 (m, 3H, H₆H_{3'}H_{5'}), 7.71 (m, 3H, H₇H_{2"}H_{6"}), 7.35 (m, 2H, H_{3"}H_{5"}), 2.13 (q, *J =* 7.6 Hz, 2H, H_{7"}), 1.23 (t, *J =* 7.6 Hz, 3H, H_{8"}).
¹³C NMR (75 MHz, DMSO-d₆) δ 167.8 (COO-), 155.3 (C₂), 148.4 (C₈ₐ), 143.6 (C₄), 141.4 (C_{4'}), 138.0 (C_{4"}), 136.6 (C_{1"}), 136.5 (C_{1'}), 130.2 (C₈), 129.7 (C₆), 128.4 (C_{2"}C_{6"}), 127.7 (C_{2'}C_{6'}), 127.6 (C₇), 126.9 (C_{3'}C_{5'}), 126.6 (C_{3"}C_{5"}), 125.5 (C₅), 123.4 (C₄ₐ), 118.9 (C₃), 27.8 (C_{7"}), 15.5 (C_{8"}).
HPLC (Sunfire C18, 60-95% of acetonitrile in water, 10 min): t_{R} = 5.00 min.
LC-MS (m/z): 354.2 ([M+H]⁺).

### Example 20: 2-(4'-butyl-[1,1'-biphenyl]-4-yl)quinoline-4-carboxylic acid

The product was prepared according to the methods described above and obtained as a yellow solid with a yield of 85%.
m.p.: 251.3 °C
¹H NMR (300 MHz, DMSO-d₆) δ 8.76 (dd, *J =* 8.5, 0.7 Hz, 1H, H₅), 8.46 (s, 1H, H₃), 8.28 (m, 3H, H₈H_{2'}H_{6'}), 7.79 (m, 3H, H₆H_{3'}H_{5'}), 7.63 (m, 3H, H₇H_{2"}H_{6"}), 7.30 (d, *J =* 8.0 Hz, 2H, H_{3"}H_{5"}), 4.09 (s, 3H, H₉), 2.08 (t, *J =* 7.7 Hz, 2H, H_{7"}), 1.66 (p, *J =* 7.6 Hz, 2H, H_{8"}), 1.40 (h*, J* = 7.5 Hz, 2H, H_{9"}), 0.97 (t, *J* = 7.3 Hz, 2H, H_{10"}).
¹³C NMR (75 MHz, DMSO-d₆) δ 167.7 (COO-), 155.4 (C₂), 148.9 (C₈ₐ), 1432.2 (C₄), 141.5 (C_{4'}), 137.7 (C_{1"}), 136.7 (C_{4"}), 136.6 (C_{1'}), 130.2 (C₈), 129.8 (C₆), 129.0 (C_{2"}C_{6"}), 127.7 (C_{2'}C_{6'}), 127.7 (C₇), 127.0 (C_{3'}C_{5'}), 126.6 (C_{3"}C_{5"}), 125.4 (C₅), 123.5 (C₄ₐ), 119.0 (C₃), 34.5 (C_{7"}), 33.1 (C_{8"}), 21.7 (C_{9"}), 13.8 (C_{10"}).
HPLC (Sunfire C18, 60-95% of acetonitrile in water, 10 min): t_{R} = 7.65 min.
LC-MS (m/z): 382.4 ([M+H]⁺).

### Example 21: 2-(4'-(tert-butyl)-[1,1'-biphenyl]-4-yl)quinoline-4-carboxylic acid

The product was prepared according to the methods described above and obtained as a yellow solid with a yield of 92%.
m.p.: 276.7 °C
¹H NMR (300 MHz, DMSO-d₆) δ 8.66 (d, *J =* 8.4 Hz, 1H, H₅), 8.47 (s, 1H, H₃), 8.39 (m, 2H, H_{2'}H_{6'}), 8.18 (d, *J =* 8.7 Hz, 1H, H₈), 7.85 (m, 3H, H₆H_{3'}H_{5'}), 7.71 (m, 3H, H₇H_{2"}H_{6"}), 7.53 (m, 2H, H_{3"}H_{5"}), 1.34 (s, 9H, H_{*t*Bu}).
¹³C NMR(75 MHz, DMSO-d₆) δ 167.6 (COO-), 155.3 (C₂), 150.4 (C₈ₐ), 148.4 (C₄), 141.4 (C_{4'}), 137.9 (C_{4"}), 136.6 (C_{1"}), 136.4 (C_{1'}), 130.2 (C₈), 129.7 (C₆), 127.7 (C_{2"}C_{6"}), 127.7 (C₇), 126.9 (C_{2'}C_{6'}), 126.4 (C_{3'}C_{5'}), 125.8 (C_{3"}C_{5"}), 125.4 (C₅), 123.4 (C₄ₐ), 118.9 (C₃), 34.3 (C_{7"}), 31.0 (C_{*t*Bu}).
HPLC (Sunfire C18, 60-95% of acetonitrile in water, 10 min): t_{R} = 6.82 min.

### Example 22: 2-(4'-acetyl-[1,1'-biphenyl]-4-yl)quinoline-4-carboxylic acid

The product was prepared according to the methods described above and obtained as a white solid with a yield of 95%.
m.p.: 267.6 °C
¹H NMR (300 MHz, DMSO-d₆) δ 14.1 (s, 1H, -COOH), 8.66 (dd, *J =* 8.6, 1.4 Hz, 1H, H₅), 8.53 (s, 1H, H₃), 8.45 (m, 2H, H_{2'}H_{6'}), 8.19 (m, 1H, H₈), 8.09 (m, 2H, H_{3"}H_{5"}), 7.96 (m, 4H, H_{3'}H_{5'}H_{2"}H_{6"}), 7.87 (ddd, *J =* 8.4, 6.9, 1.5 Hz, 1H, H₆), 7.72 (ddd, *J =* 8.3, 6.8, 1.4 Hz, 1H, H₇), 2.64 (s, 3H, H_{7"}).
¹³C NMR (75 MHz, DMSO-d₆) δ 199.9 (C_{7"}), 169.9 (-COOH), 157.5 (C₂), 150.7 (C₈ₐ), 145.9 (C₄), 142.5 (C_{4'}), 140.2 (C_{1"}), 140.0 (C_{4"}), 138.3 (C_{1'}), 132.6 (C₈), 132.1 (C₆), 131.3 (C_{2"}C_{6"}), 130.2 (C_{2'}C_{6'}), 130.2 (C₇), 129.9 (C_{3"}C_{5"}), 129.3 (C_{3'}C_{5'}), 127.8 (C₅), 125.9 (C₄ₐ), 121.4 (C₃), 29.1 (C_{8"}).
HPLC (Sunfire C18, 60-95% of acetonitrile in water, 10 min): t_{R} = 1.61 min.
LC-MS (m/z): 368.1 ([M+H]⁺).

### Example 23: 2-(3',4'-dichloro-[1,1'-biphenyl]-4-yl)quinoline-4-carboxylic acid

The product was prepared according to the methods described above and obtained as a yellow solid with a yield of 78%.
m.p.: 226.8 °C
¹H NMR (300 MHz, DMSO-d₆) δ 8.64 (dd, *J =* 8.6, 1.4 Hz, 1H, H₅), 8.37 (m, 3H, H₃H_{2'}H_{6'}), 8.11 (d, *J =* 8.5 Hz, 1H, H₈), 8.02 (d, *J =* 1.9 Hz, 1H, H_{2"}), 7.89 (m, 2H, H_{3'}H_{5'}), 7.76 (m, 3H, H₆H₇H_{5"}), 7.62 (m, 1H, H_{6"}).
¹³C NMR (75 MHz, DMSO-d₆) δ 168.6 (COO-), 154.9 (C₂), 148.3 (C₈ₐ), 139.9 (C₄), 138.4 (C_{4'}), 138.1 (C_{1"}), 131.8 (C_{1'}), 131.0 (C_{3"}), 130.5 (C_{4"}), 129.7 (C_{5"}), 129.4 (C_{2"}), 128.4 (C₇), 127.7 (C_{2'}C_{6'}), 127.2 (C_{3'}C_{5'}), 126.9 (C_{6"}), 126.9 (C₈), 126.9 (C₆), 126.2 (C₅), 124.0 (C₄ₐ), 117.7 (C₃).
HPLC (Sunfire C18, 60-95% of acetonitrile in water, 10 min): t_{R} = 7.88 min.
LC-MS (m/z): 394.2 ([M]⁺).

### Example 24: 2-(3'-(trifluoromethoxy)-[1,1'-biphenyl]-4-yl)quinoline-4-carboxylic acid

The product was prepared according to the methods described above and obtained as a yellow solid with a yield of 81%.
m.p.: 226.5 °C
¹H NMR (300 MHz, DMSO-d₆) δ 8.66 (dd, *J* = 8.6, 1.3 Hz, 1H, H₅), 8.53 (s, 1H, H₃), 8.44 (m, 2H, H_{2'}H_{6'}), 8.20 (dd, *J =* 8.6, 1.2 Hz, 1H, H₈), 7.95 (m, 2H, H_{3'}H_{5'}), 7.86 (m, 2H, H₆H₇), 7.77 (s, 1H, H_{2"}), 7.72 (m, 1H, H_{6"}), 7.66 (t, *J =* 8.0 Hz, 1H, H_{5"}), 7.43 (dt, *J* = 8.2, 2.3 Hz, 1H, H_{4"}).
¹³C NMR (75 MHz, DMSO-d₆) δ 167.6 (COO-), 155.1 (C₂), 149.0 (C_{3"}), 148.4 (C₄), 141.7 (C₈ₐ), 139.7 (C_{4'}), 137.8 (C_{1"}), 137.6 (C_{1'}), 131.0 (C_{5"}), 130.3 (C₄ₐ), 129.8 (C₅), 127.9 (C_{2'}C_{6'}), 127.9 (C₇), 127.4 (C_{3'}C_{5'}), 125.9 (C₆), 125.4 (C₈), 123.5 (C_{6"}), 120.2 (C_{4"}), 119.8 (C_{7"}), 119.3 (C_{2"}), 119.1 (C₃).
HPLC (Sunfire C18, 60-95% of acetonitrile in water, 10 min): t_{R} = 7.88 min.
LC-MS (m/z): 410.2 ([M]⁺).

### Example 25: 2-([1,1'-biphenyl]-3-yl)quinoline-4-carboxylic acid

The product was prepared according to the methods described above and obtained as a white solid with a yield of 92%.
m.p.: 113.0 °C
¹H NMR (300 MHz, DMSO-d₆) δ 8.65 (dd, *J* = 8.5, 1.5 Hz, 1H, H_{2'}), 8.47 (t, *J =* 1.8 Hz, 1H, H_{3'}), 8.23 (m, 2H, H₅H₇), 8.08 (dd, *J =* 8.5, 1.3 Hz, 1H, H₈), 7.81 (m, 1H, H_{6'}), 7.77 (m, 2H, H_{2"}H_{6"}), 7.73 (m, 1H, H₆), 7.65 (t, J = 7.7 Hz, 1H, H_{5'}), 7.56 (m, 1H, H_{4'}), 7.51 (m, 2H, H_{3"}H_{5"}), 7.43 (m, 1H, H_{4"}).
¹³C NMR(75 MHz, DMSO-d₆) δ 168.7 (COO-), 155.5 (C₂), 148.2 (C₈ₐ), 140.8 (C₄), 140.0 (C_{4"}), 139.5 (C_{3'}), 129.5 (C_{1'}), 129.3 (C_{1"}), 129.2 (C₇), 129.0 (C_{2"}C_{6"}), 127.8 (C_{5'}), 127.6 (C_{4'}), 127.0 (C₈), 126.9 (C_{3"}C_{5"}), 126.2 (C₆), 126.1 (C₅), 126.1 (C_{2'}), 125.3 (C_{6'}), 124.4 (C₄ₐ), 117.0 (C₃).
HPLC (Sunfire C18, 60-95% of acetonitrile in water, 10 min): t_{R} = 2.38 min.
LC-MS (m/z): 326.3 ([M+H]⁺).

### Example 26: 2-(4'-ethyl-[1,1'-biphenyl]-3-yl)quinoline-4-carboxylic acid

The product was prepared according to the methods described above and obtained as a white solid with a yield of 88%.
m.p.: 246.7 °C
¹H NMR (300 MHz DMSO-d₆) δ 13.98 (s, 1H, COOH), 8.64 (dd, *J =* 8.7, 1.4 Hz, 1H, H₅), 8.56 (s, 1H, H₃), 8.51 (s, 1H, H_{2'}), 8.27 (dt, *J* = 7.9, 1.3 Hz, 1H, H₈), 8.20 (d, *J =* 7.8 Hz, 1H, H_{6'}), 7.87 (m, 1H, H₇), 7.81 (m, 1H, H_{4'}), 7.73 (m, 2H, H_{2"}H_{6"}), 7.67 (m, 2H, H₆H_{5'}), 7.37 (m, 2H, H_{3"}H_{5"}), 2.68 (q, *J =* 7.6 Hz, 2H, H_{7"}), 1.24 (t, *J =* 7.6 Hz, 3H, H_{8"}).
¹³C NMR(75 MHz, DMSO-d₆) δ 167.7 (COO-), 155.8 (C₂), 148.3 (C₈ₐ), 143.3 (C₄), 140.9 (C_{4"}), 138.5 (C_{3'}), 137.8 (C_{1'}), 137.3 (C_{1"}), 130.2 (C_{4'}), 129.8 (C_{2'}), 129.6 (C_{5'}), 127.7 (C_{2"}C_{6"}), 128.1 (C₇), 127.8 (C₈), 126.9 (C_{3"}C_{5"}), 126.1 (C₆), 125.3 (C₅), 125.3 (C_{6'}), 123.4 (C₄ₐ), 119.3 (C₃), 27.8 (C_{7"}), 15.6 (C_{8"}).
HPLC (Sunfire C18, 50-95% of acetonitrile in water, 10 min): t_{R} = 7.00 min.
LC-MS (m/z): 354.3 ([M+H]⁺).

### Example 27: 2-(4'-butyl-[1,1'-biphenyl]-3-yl)quinoline-4-carboxylic acid

The product was prepared according to the methods described above and obtained as a yellow solid with a yield of 80%.
m.p.: 208.0 °C
¹H NMR (300 MHz, DMSO-d₆) δ 13.98 (s, 1H, COOH), 8.64 (dd, *J =* 7.7, 0.9 Hz, 1H, H₅), 8.56 (s, 1H, H₃), 8.51 (t, *J* = 1.7 Hz, 1H, H_{2'}), 8.27 (dt, *J* = 7.9, 1.3 Hz, 1H, H_{6'}), 8.21 (d, *J =* 7.9 Hz, 1H, H₈), 7.87 (m, 1H, H₇), 7.81 (m, 1H, H_{4'}), 7.73 (m, 3H, H₆H_{2"}H_{6"}), 7.66 (t, *J =* 7.8 Hz, 1H, H_{5'}), 7.31 (d, *J =* 8.2 Hz, 2H, H_{3"}H_{5"}), 2.66 (t, *J =* 7.6 Hz, 2H, H_{7"}), 1.62 (m, 2H, H_{5"}), 1.35 (sext, *J* = 7.3 Hz, 2H, H_{9"}), 0.93 (t, *J =* 7.3 Hz, 3H, H_{10"}).
¹³C NMR(75 MHz, DMSO-d₆) δ 168.0 (COO-), 156.1 (C₂), 148.7 (C₈ₐ), 142.3 (C₄), 141.2 (C_{4"}), 138.9 (C_{3'}), 138.2 (C_{1'}), 137.6 (C_{1"}), 130.6 (C₄ₐ), 130.1 (C_{2'}), 129.9 (C_{5'}), 129.3 (C_{2"}C_{6"}), 128.4 (C₇), 128.1 (C₈), 127.1 (C_{3"}C_{5"}), 126.4 (C₆), 125.7 (C₅), 125.6 (C_{6'}), 123.8 (C_{4'}), 119.6 (C₃), 34.8 (C_{7"}), 33.4 (C_{8"}), 22.1 (C_{9"}), 14.1 (C_{10"}).
HPLC (Sunfire C18, 50-95% of acetonitrile in water, 10 min): t_{R} = 9.43 min.
LC-MS (m/z): 382.4 ([M+H]⁺).

### Example 28: 2-(4'-(tert-butyl)-[1,1'-biphenyl]-3-yl)quinoline-4-carboxylic acid

The product was prepared according to the methods described above and obtained as a yellow solid with a yield of 97%.
m.p.: 234.2 °C
¹H NMR (300 MHz, DMSO-d₆) δ 14.03 (s, 1H, COOH), 8.65 (dd, *J =* 8.7, 1.4 Hz, 1H, H₅), 8.56 (s, 1H, H₃), 8.51 (t, *J =* 1.8 Hz, 1H, H_{2'}), 8.27 (dt, *J* = 7.8, 1.4 Hz, 1H, H_{6'}), 8.20 (dd, *J =* 8.7, 1.3 Hz, 1H, H₈), 7.87 (ddd, *J =* 8.4, 6.8, 1.5 Hz, 1H, H₇), 7.80 (dt, *J =* 7.8, 1.4 Hz, 1H, H_{4'}), 7.73 (m, 3H, H₆H_{2"}H_{6"}), 7.67 (t, *J =* 7.7 Hz, H_{5'}), 7.54 (d, *J =* 8.4 Hz, 2H, H_{3"}H_{5"}), 1.34 (s, 9H, H*_{tBu}*).
¹³C NMR (75 MHz, DMSO-d6) δ 167.6 (COO-), 155.7 (C₂), 150.1 (C₈ₐ), 148.3 (C₄), 140.9 (C_{4"}), 138.5 (C_{3'}), 137.8 (C_{1'}), 137.1 (C_{1"}), 130.2 (C₄ₐ), 129.8 (C_{2'}), 129.6 (C_{5'}), 128.1 (C₇), 127.8 (C₈), 126.6 (C_{2"}C_{6"}), 126.1 (C₆), 125.7 (C_{3"}C_{5"}), 125.3 (C₅), 125.3 (C_{6'}), 123.4 (C_{4'}), 119.3 (C₃), 34.2 (C_{7"}), 31.1 (C_{8"}).
HPLC (Sunfire C18, 50-95% of acetonitrile in water, 10 min): t_{R} = 8.71 min.

### Example 29: 2-(4'-acetyl-[1,1'-biphenyl]-3-yl)quinoline-4-carboxylic acid

The product was prepared according to the methods described above and obtained as a yellow solid with a yield of 92%.
¹H NMR (300 MHz Chloroform-d) δ 8.64 (d, *J =* 8.5, Hz, 1H, H₅), 8.60 (s, 2H, H₃H_{2'}), 8.35 (d, *J* = 7.8 Hz, 1H, H_{6'}), 8.24 (d, *J* = 8.3 Hz, 1H, H₈), 8.09 (d, *J* = 8.4 Hz, 2H, H_{3"}H_{5"}), 7.97 (d, *J =* 8.4 Hz, 2H, H_{2"}H_{6"}), 7.74 (m, 2H, H₇H_{4'}), 7.73 (m, 2H, H₆H_{5'}), 2.64 (s, 3H, H_{8"}),
¹³C NMR (75 MHz, Chloroform-d) δ 198.4 (C_{7"}), 168.4 (COO-), 156.3 (C₂), 148.8 (C₈ₐ), 145.0 (C₄), 140.5 (C_{3'}), 139.3 (C_{1"}), 139.0 (C_{4"}), 136.7 (C_{1'}), 131.3 (C₇), 130.6 (C₈C_{4'}), 130.3 (C_{4'}), 129.6 (C_{2"}C_{6"}), 129.5 (C_{2'}), 128.8 (C_{6'}), 128.1 (C_{5'}), 128.1 (C_{3"}C_{5"}), 126.7 (C₆), 126.2 (C₅), 124.3 (C₄ₐ), 120.2 (C₃), 27.6 (C_{8"}).
HPLC (Sunfire C18, 60-95% of acetonitrile in water, 10 min): t_{R} = 1.59 min.
LC-MS (m/z): 368.2 ([M+H]⁺).

### Example 30: 2-(3'-(trifluoromethoxy)-[1,1'-biphenyl]-3-yl)quinoline-4-carboxylic acid

The product was prepared according to the methods described above and obtained as a white solid with a yield of 89%.
m.p.: 194.6 °C
¹H NMR (300 MHz, DMSO-d6) δ 8.63 (dd, *J =* 8.6, 1.4 Hz, 1H, H₅), 8.60 (s, 1H, H₃), 8.55 (d, *J =* 1.9 Hz, 1H, H_{2'}), 8.36 (dt, *J =* 7.9, 1.4 Hz, 1H, H₇), 8.21 (d, *J =* 8.4 Hz, 1H, H₈), 7.88 (m, 2H, H₆H_{2"}H_{6"}), 7.84 (d, *J =* 8.3 Hz, 1H, H_{6'}), 7.70 (m, 3H, H_{5'}H_{3"}H_{5"}), 7.44 (m, 1H, H_{4'}).
¹³C NMR (75 MHz, DMSO-d6) δ 168.6 (COO-), 156.1 (C₂), 152.9 (C_{3"}), 144.7 (C₈ₐ), 144.3 (C₄), 140.2 (C_{3'}), 138.6 (C_{1"}), 138.1 (C_{1'}), 130.6 (C_{2'}), 129.8 (C_{5"}), 129.2 (C₄ₐ), 129.1 (C_{5'}), 127.6 (C₅), 127.2 (C₆), 127.1 (C₇), 126.0 (C₈), 125.4 (C_{6'}), 125.0 (C_{4'}), 125.0 (C_{6"}), 122.3 (C_{7"}), 119.7 (C_{4"}), 119.0 (C_{2"}), 117.8 (C₃).
HPLC (Sunfire C18, 60-95% of acetonitrile in water, 10 min): t_{R} = 4.38 min.
LC-MS (m/z): 410.3 ([M+H]⁺).

### Example 31: 2-(3-bromophenyl)quinoline-4-carboxylic acid

The product was prepared according to the methods described above and obtained as a brown solid with a yield of 96%.
¹H NMR (300 MHz, DMSO-d6) δ 14.01 (s, 1H, -COOH) 8.64 (dd, *J =* 8.5, 1.3 Hz, 1H, H₅), 8.50 (m, 2H, H₃H₈), 8.30 (td, *J =* 7.9, 1.3 Hz, 1H, H_{6'}), 8.20 (dd, *J =* 8.6, 1.2 Hz, 1H, H_{2'}), 7.87 (m, 1H, H₇), 7.73 (m, 2H, H₆H_{4'}), 7.54 (t, *J =* 7.9 Hz, 1H, H_{5'}).
¹³C NMR (75 MHz, DMSO-d6) δ 167.5 (COO-), 154.1 (C₂), 142.2 (C₈ₐ), 140.1 (C₄), 138.0 (C_{1'}), 132.6 (C_{4'}), 1381.1 (C_{2'}), 130.4 (C_{5'}), 129.8 (C₄ₐ), 129.7 (C₅), 128.1 (C₆), 126.3 (C₇), 125.3 (C₈), 123.6 (C_{6'}), 122.5 (C_{3'}), 119.0 (C₃).
HPLC (Sunfire C18, 60-95% of Acetonitrile in water, 10 min): t_{R} = 1.95 min.
LC-MS (m/z): 328.2 ([M]⁺).

### Example 32: 7-(4-ethylphenyl)-2-(4-methoxyphenyl)quinoline-4-carboxylic acid

The product was prepared according to the methods described above and obtained as a yellow solid with a yield of 77%.
m.p.: 247.3 °C
¹H NMR (300 MHz, DMSO-d6) δ 8.70 (d, *J =* 8.8 Hz, 1H, H₅), 8.40 (s, 1H, H₃), 8.34 (d, *J* = 1.9 Hz, 1H, H₈), 8.29 (m, 2H, H_{2"}H_{6"}), 8.00 (dd, *J =* 8.9, 2.0 Hz, 1H, H₆), 7.82 (m, 2H, H_{2'}H_{6'}), 7.38 (d, *J =* 8.2 Hz, 2H, H_{3"}H_{5"}), 7.13 (m, 2H, H_{3'}H_{5'}), 3.86 (s, 3H, H_{7'}), 2.68 (q, *J =* 7.6 Hz, 2H, H_{7"}), 1.24 (t, *J =* 7.6 Hz, 3H, H_{8"}).
¹³C NMR (75 MHz, DMSO-d6) δ 168.0 (-COO), 161.3 (C_{4'}), 156.2 (C₂), 149.2 (C₈ₐ), 144.3 (C₄), 141.7 (C_{4"}), 137.4 (C₇), 136.6 (C_{1"}), 130.6 (C_{1'}), 129.0 (C_{2"}C_{6"}), 128.9 (C_{2'}C_{6'}), 127.4 (C_{3"}C_{5"}), 126.7 (C₄ₐ), 126.3 (C₆), 126.3 (C₅), 122.5 (C₈), 118.9 (C₃), 114.7 (C_{3'}C_{5'}), 55.7 (C_{7'}), 28.2 (C_{7"}), 15.9 (C_{8"}).
HPLC (Sunfire C18, 60-95% of Acetonitrile in water, 10 min): t_{R} = 2.36 min.
LC-MS (m/z): 384.3 ([M+H]⁺).

### Example 33: 7-(4-butylphenyl)-2-(4-methoxyphenyl)quinoline-4-carboxylic acid

The product was prepared according to the methods described above and obtained as a yellow solid with a yield of 89%.
m.p.: 215.1 °C
¹H NMR (300 MHz, DMSO-d6) δ 8.70 (d, *J =* 8.9 Hz, 1H, H₅), 8.41 (s, 1H, H₃), 8.36 (d, *J =* 1.9 Hz, 1H, H₈), 8.30 (m, 2H, H_{2'}H_{6'}), 8.01 (dd, *J =* 8.9, 2.0 Hz, 1H, H₆), 7.82 (m, 2H, H_{2"}H_{6"}), 7.37 (m, 2H, H_{3"}H_{5"}), 7.14 (m, 2H, H_{3'}H_{5'}), 3.86 (s, 3H, H_{7'}), 2.66 (t, *J =* 7.6 Hz, 2H, H_{7"}), 1.61 (m, 2H, H_{8"}), 1.35 (m, 2H, H_{9"}), 0.92 (t, *J =* 7.3 Hz, 3H, H_{10"}).
¹³C NMR (75 MHz, DMSO-d6) δ 167.6 (-COO), 161.0 (C_{4'}), 155.8 (C₂), 148.7 (C₈ₐ), 142.6 (C_{4"}), 141.5 (C₇), 137.3 (C_{1"}), 136.1 (C₄), 130.1 (C_{1'}), 129.1 (C_{3"}C_{5"}), 128.8 (C_{2'}C_{6'}), 127.0 (C_{2"}C_{6"}), 126.4 (C₆), 126.0 (C₄ₐ), 125.7 (C₈), 122.2 (C₅), 118.6 (C₃), 114.4 (C_{3'}C_{5'}), 55.3 (C_{7'}), 34.4 (C_{7"}), 33.0 (C_{8"}), 21.8 (C_{9"}), 13.8 (C_{10"}).
HPLC (Sunfire C18, 60-95% of Acetonitrile in water, 10 min): t_{R} = 6.90 min.
LC-MS (m/z): 412.4 ([M+H]⁺).

### Example 34: 7-(4-(tert-butyl)phenyl)-2-(4-methoxyphenyl)quinoline-4-carboxylic acid

The product was prepared according to the methods described above and obtained as a yellow solid with a yield of 87%.
m.p.: 291.5 °C
¹H NMR (300 MHz, DMSO-d₆) δ 8.70 (d, *J* = 8.9 Hz, 1H, H₅), 8.40 (s, 1H, H₃), 8.34 (d, *J* = 1.8 Hz, 1H, H₈), 8.29 (d, *J* = 8.7 Hz, 2H, H₂H₆), 7.99 (dd, *J* = 8.9, 1.9 Hz, 1H, H₆), 7.83(d, *J* = 8.2 Hz, 2H, H_{2"}H_{6"}), 7.55 (d, *J* = 8.1 Hz, 2H, H_{3"}H_{5"}), 7.13 (d, *J* = 8.8 Hz, 2H, H_{3'}H_{5'}), 3.86 (s, 3H, H_{7'}), 1.33 (s, 3H, H_{8"}).
¹³C NMR (75 MHz, DMSO-d₆) δ 167.6 (-COO), 160.9 (C₂), 155.9 (C_{4"}), 150.8 (C₈ₐ), 148.9 (C₄), 141.2 (C_{4'}), 137.2 (C₇), 136.0 (C_{1'}), 130.3 (C_{1"}), 128.7 (C_{2"}C_{6"}), 126.8 (C_{3'}C_{5'}), 126.3 (C₄ₐ), 126.0 (C₈), 126.0 (C₆), 125.9 (C_{2'}C_{6'}), 122.2 (C₅), 118.5 (C₃), 114.4 (C_{3"}C_{5"}), 55.3 (C₇), 34.3 (C_{7"}), 31.0 (C_{8"}).
HPLC (Sunfire C18, 60-95% of Acetonitrile in water, 10 min): t_{R} = 3.60 min.
LC-MS (m/z): 412.4 ([M+H]⁺).

### Example 35: 7-(4-acetylphenyl)-2-(4-methoxyphenyl)quinoline-4-carboxylic acid

The product was prepared according to the methods described above and obtained as a yellow solid with a yield of 65%.
m.p.: 233.8 °C
¹H NMR (300 MHz, DMSO-d₆) δ 8.75 (d, *J* = 8.9 Hz, 1H, H₅), 8.47 (d, *J* = 2.0 Hz, 1H, H₈), 8.45 (s, 1H, H₃), 8.31 (m, 2H, H_{3"}H_{5"}), 8.08 (m, 5H, H₆ H_{2'} H_{6'}H_{2"} H_{6"}), 7.14 (m, 2H, H_{3'}H_{5'}), 3.87 (s, 3H, H_{7'}), 2.65 (s, 3H, H_{8"}).
¹³C NMR (75 MHz, DMSO-d₆) δ 197.5 (C_{7"}), 167.5 (-COO), 161.0 (C_{4'}), 156.1 (C₂), 148.7 (C₈ₐ), 143.2 (C₄), 140.1 (C_{1"}), 137.1 (C_{4"}), 136.2 (C₇), 130.1 (C_{1'}), 129.0 (C_{2"}C_{6"}) 128.7 (C_{2'}C_{6'}), 127.4 (C_{3"}C_{5"}), 127.1 (C₄ₐ), 126.2 (C₈), 126.2 (C₆), 122.8 (C₅), 119.1 (C₃), 114.4 (C_{3'}C_{5'}), 55.3 (C₇), 26.8 (C_{8"}).
HPLC (Sunfire C18, 60-95% of Acetonitrile in water, 10 min): t_{R} = 1.53 min.
LC-MS (m/z): 398.3 ([M+H]⁺).

### Example 36: 7-(3,4-dichlorophenyl)-2-(4-methoxyphenyl)quinoline-4-carboxylic acid

The product was prepared according to the methods described above and obtained as a yellow solid with a yield of 90%.
m.p.: 207.2 °C
¹H NMR (300 MHz, DMSO-d₆) δ 8.73 (d, *J* = 8.8 Hz, 1H, H₅), 8.35 (d, *J* = 2.0 Hz, 1H, H₈), 8.25 (m, 3H, H₃H_{2'}H_{6'}), 8.18 (d, *J* = 2.2 Hz, 1H, H_{2"}), 7.92 (m, 2H, H_{5"}H_{6"}), 7.78 (d, *J* = 8.4 Hz, 1H, H₆), 7.11 (m, 2H, H_{3'}H_{5'}), 3.90 (s, 3H, H_{7'}).
¹³C NMR (75 MHz, DMSO-d₆) δ 167.9 (-COO), 160.4 (C_{4'}), 155.7 (C₂), 148.2 (C₈ₐ), 139.6 (C₄), 137.8 (C_{1"}), 131.6 (C₇), 133.2 (C_{1'}C_{5"}), 130.5 (C₄ₐ), 130.5 (C_{3"}), 128.6 (C₈), 128.3 (C_{2'}C_{6'}), 127.1 (C_{4"}), 127.0 (C_{6"}), 126.4 (C_{2"}) 124.6 (C₆), 123.2 (C₅), 117.3 (C₃), 114.0 (C_{3'}C_{5'}), 55.0 (C_{7'}).
HPLC (Sunfire C18, 60-95% of Acetonitrile in water, 10 min): t_{R} = 5.72 min.
LC-MS (m/z): 424.1 ([M]⁺).

### Example 37: 2-(4-methoxyphenyl)-7-(3-(trifluoromethoxy)phenyl)quinoline-4-carboxylic acid

The product was prepared according to the methods described above and obtained as a yellow solid with a yield of 88%.
m.p.: 292.1 °C
¹H NMR (300 MHz, DMSO-d₆) δ 8.74(d, *J* = 8.9 Hz, 1H, H₅), 8.44 (s, 1H, H₃), 8.43 (d, *J* = 2.1 Hz, 1H, H₈), 8.30 (m, 2H, H_{2'}H_{6'}), 8.04 (dd, *J* = 8.9, 2.0 Hz, 1H, H₆), 7.97 (dt, *J* = 8.0, 1.2 Hz, 1H, H_{6"}), 7.90 (s, 1H, H_{2"}), 7.68 (t, *J* = 8.0, 1H, H_{5"}), 7.46 (ddt, *J* = 8.2, 2.3, 1.1 Hz, 1H, H_{4"}), 7.13 (m, 2H, H_{3'}H_{5'}), 3.86 (s, 3H, H_{7'}).
¹³C NMR (75 MHz, DMSO-d₆) δ 167.5 (-COO), 161.0 (C_{4'}), 156.1 (C₂), 149.1 (C_{3"}), 148.7 (C₈ₐ), 141.2 (C₄), 139.6 (C_{1"}), 137.1 (C₇), 131.1 (C_{1'}), 130.2 (C₈), 128.7 (C_{2'}C_{6'}), 127.0 (C_{5"}), 126.3 (C₆), 126.3 (C₅), 126.2 (C_{6"}), 122.7 (C₄ₐ), 121.8 (C_{7"}), 120.6 (C_{4"}), 119.8 (C_{2"}), 119.0 (C₃), 114.3 (C_{3'}C_{5'}), 55.3 (C_{7'}).
HPLC (Sunfire C18, 60-95% of Acetonitrile in water, 10 min): t_{R} = 4.64 min.
LC-MS (m/z): 440.2 ([M+H]⁺).

### Example 38: 2-(4-methoxyphenyl)-7-(4-propoxyphenyl)quinoline-4-carboxylic acid

The product was prepared according to the methods described above and obtained as a yellow solid with a yield of 100%.
m.p.: 251.5 °C
¹H NMR (300 MHz, DMSO-d₆) δ 8.67 (d, *J* = 8.9 Hz, 1H, H₅), 8.37 (s, 1H, H₃), 8.28 (m, 3H, H₈H_{2'}H_{6'}), 7.97 (dd, *J* = 8.9, 2.0 Hz, 1H, H₆), 7.84 (m, 2H, H_{2"}H_{6"}), 7.10 (m, 4H, H_{3'}H_{5'}H_{3"}H_{5"}), 4.00 (t, *J* = 6.5 Hz, 2H, H_{7"}), 3.86 (s, 3H, H_{7'}), 1.76 (h, *J* = 7.1 Hz, 2H, H_{8"}), 1.00 (t, *J* = 7.4 Hz, 3H, H_{9"}).
¹³C NMR (75 MH7z, DMSO-d₆) δ 167.7 (-COO), 160.9 (C_{4'}), 159.0 (C_{4"}), 155.8 (C₂), 149.0 (C₈ₐ), 141.0 (C₄), 137.4 (C₇), 130.9 (C_{1"}), 130.4 (C_{1'}), 128.7 (C_{2'}C_{6'}), 128.3 (C_{2"}C_{6"}), 126.0 (C₈), 126.0 (C₆), 125.4 (C₅), 121.9 (C₄ₐ), 118.0 (C₃), 115.0 (C_{3"}C_{5"}), 114.3 (C_{3'}C_{5'}), 69.0 (C_{7"}), 55.3 (C₇), 22.0 (C_{8"}), 10.4 (C_{9"}).
HPLC (Sunfire C18, 60-95% of Acetonitrile in water, 10 min): t_{R} = 3.53 min.
LC-MS (m/z): 414.4 ([M+H]⁺).

### Example 39: 2-(4-methoxyphenyl)-7-(3,4,5-trimethoxyphenyl)quinoline-4-carboxylic acid

The product was prepared according to the methods described above and obtained as a yellow solid with a yield of 71%.
m.p.: 279.7 °C
¹H NMR (300 MHz, DMSO-d₆) δ 8.69 (d, *J* = 8.9 Hz, 1H, H₈), 8.41 (m, 2H, H₃H₈), 8.30 (m, 2H, H_{2'}H_{6'}), 8.05 (dd, *J* = 8.9, 2.0 Hz, 1H, H₆), 7.14 (m, 4H, H_{3'}H_{5'}H_{2"}H_{6"}), 3.93 (s, 6H, H_{7"}H_{9"}), 3.86 (s, 3H, H_{8"}), 3.74 (s, 3H, H_{7'}).
¹³C NMR (75 MHz, DMSO-d₆) δ 167.6 (-COO), 161.0 (C_{4'}), 155.9 (C₂), 153.4 (C_{3"}C_{5"}), 148.7 (C₈ₐ), 141.6 (C₄), 137.8 (C_{4"}), 137.2 (C₇), 134.6 (C_{1"}), 130.2 (C_{1'}), 128.8 (C_{2'}C_{6'}), 126.7 (C₈), 126.2 (C₆), 125.8 (C₅), 122.2 (C₄ₐ), 118.6 (C₃), 114.4 (C_{3'}C_{5'}), 104.6 (C_{2"}C_{6"}), 60.1 (C_{8"}), 56.1 (C_{7"}C_{9"}), 55.3 (C_{7'}).
HPLC (Sunfire C18, 60-95% of Acetonitrile in water, 10 min): t_{R} = 1.47 min.
LC-MS (m/z): 446.3 ([M+H]⁺).

### Example 40: 2-(4-methoxyphenyl)-7-(4-morpholinophenyl)quinoline-4-carboxylic acid

The product was prepared according to the methods described above and obtained as a black solid with a yield of 98%. m.p.: n.d.
¹H NMR (300 MHz, DMSO-d₆) δ 8.66 (d, *J* = 8.9 Hz, 1H, H₅), 8.39 (m, 2H, H₃H₈), 8.29 (m, 2H, H_{2'}H_{6'}), 8.02 (dd, *J* = 8.9, 2.0 Hz, 1H, H₆), 7.81 (m, 2H, H_{2"}H_{6"}), 7.14 (m, 4H, H_{3'}H_{5'}H_{3"}H_{5"}), 3.86 (s, 3H, H_{7'}), 3.78 (t, *J* = 4.2 Hz, 4H, H_{8"}H_{9"}), 3.23 (t, *J* = 4.6 Hz, 4H, H_{7"}H_{10"}).
¹³C NMR (75 MHz, DMSO-d₆) δ 167.4 (-COO), 161.3 (C_{4'}), 155.5 (C₂), 150.5 (C_{4"}), 147.8 (C₈ₐ), 141.6 (C₄), 130.1 (C₇), 129.2 (C_{1"}), 129.2 (C_{1'}), 129.1 (C_{2"}C_{6"}), 127.8 (C_{2'}C_{6'}), 126.3 (C₈), 126.0 (C₆), 123.6 (C₅), 121.9 (C₄ₐ), 118.5 (C₃), 115.6 (C_{3"}C_{5"}), 114.4 (C_{3'}C_{5'}), 65.8 (C_{8"}C_{9"}), 55.4 (C₇), 48.2 (C_{7"}C_{9"}).
HPLC (Sunfire C18, 60-95% of Acetonitrile in water, 10 min): t_{R} = 1.30 min.
LC-MS (m/z): 441.3 ([M+H]⁺).

### Example 41: 2-(4-methoxyphenyl)-8-phenylquinoline-4-carboxylic acid.

The product was prepared according to the methods described above and obtained as a yellow solid with a yield of 67%.
m.p.: 212.3 °C
¹H NMR (300 MHz, DMSO-d₆) δ 8.58 (dd, *J* = 8.5, 1.5 Hz, 1H, H₅), 8.39 (s, 1H, H₃), 8.14 (m, 2H, H_{2"}H_{6"}), 7.82 (dd, *J* = 7.2, 1.5 Hz, 1H, H₇), 7.72 (m, 3H, H₆H_{2'}H_{6'}), 7.52 (m, 2H, H_{3"}H_{5"}), 7.43 (td, *J* = 7.2, 1.3 Hz, 1H, H_{4"}), 7.05 (m, 2H, H_{3'}H_{5'}), 3.81 (s, 3H, H_{7'}).
¹³C NMR (75 MHz, DMSO-d₆) δ 168.2 (-COO), 160.8 (C_{4'}), 154.5 (C₂), 145.5 (C₈ₐ), 140.0 (C₈), 139.3 (C₄), 139.3 (C_{1"}), 130.8 (C_{2"}C_{6"}), 130.6 (C_{1'}), 130.4 (C₇), 128.6 (C_{2'}C_{6'}), 127.6 (C_{3"}C_{5"}), 127.1 (C₆), 126.8 (C_{4"}), 125.1 (C₅), 123.5 (C₄ₐ), 117.6 (C₃), 114.3 (C_{3'}C_{5'}), 55.7 (C_{7'}).
HPLC (Sunfire C18, 60-95% of Acetonitrile in water, 10 min): t_{R} = 2.79 min.
LC-MS (m/z): 356.3 ([M+H]⁺).

### Example 42: 8-(4-ethylphenyl)-2-(4-methoxyphenyl)quinoline-4-carboxylic acid

The product was prepared according to the methods described above and obtained as a yellow solid with a yield of 97%.
m.p.: 183.8 °C
¹H NMR (300 MHz, DMSO-d₆) δ 8.55 (dd, *J* = 8.4, 1.4 Hz, 1H, H₅), 8.11 (m, 3H, H₃H_{2"}H_{6"}), 7.70 (m, 3H, H₇H_{2'}H_{6'}), 7.56 (dd, *J* = 8.4, 7.1 Hz, 1H, H₆), 7.35 (m, 2H, H_{3"}H_{5"}), 7.05 (m, 2H, H_{3'}H_{5'}), 3.82 (s, 3H, H_{7'}), 2.72 (q, *J* = 7.6 Hz, 2H, H_{7"}), 1.28 (t, *J* = 7.6 Hz, 3H, H_{8"}).
¹³C NMR (75 MHz, DMSO-d₆) δ 169.1 (-COO), 160.4 (C_{4'}), 154.4 (C₂), 145.4 (C₈ₐ), 142.3 (C₈), 139.9 (C_{4"}), 137.1 (C₄), 131.3 (C_{1"}), 130.8 (C_{1'}), 130.8 (C_{2"}C_{6"}), 129.7 (C₇), 128.4 (C_{2'}C_{6'}), 126.9 (C_{3"}C_{5"}), 126.2 (C₆), 125.5 (C₅), 124.3 (C₄ₐ), 115.7 (C₃), 114.2 (C_{3'}C_{5'}), 55.5 (C_{7'}), 27.9 (C_{7"}), 15.6 (C_{8"}).
HPLC (Sunfire C18, 60-95% of Acetonitrile in water, 10 min): t_{R} = 4.79 min.
LC-MS (m/z): 384.3 ([M+H]⁺).

### Example 43: 8-(4-butylphenyl)-2-(4-methoxyphenyl)quinoline-4-carboxylic acid

The product was prepared according to the methods described above and obtained as a yellow solid with a yield of 98%.
m.p.: 184.2 °C
¹H NMR (300 MHz, DMSO-d₆) δ 8.55 (m, 1H, H₅), 8.42 (s, 1H, H₃), 8.16 (m, 2H, H_{2"}H_{6"}), 7.82 (m, 1H, H₇), 7.69 (m, 3H, H₆H_{2'}H_{6'}), 7.33 (m, 2H, H_{3"}H_{5"}), 7.05 (m, 2H, H_{3'}H_{5'}), 3.82 (s, 3H, H_{7'}), 2.68 (t, *J* = 7.9 Hz, 2H, H_{7"}), 1.64 (m, 2H, H_{8"}), 1.38 (m, 2H, H_{9"}), 0.94 (m, 3H, H_{10"}).
¹³C NMR (75 MHz, DMSO-d₆) δ 168.0 (-COO), 160.8 (C_{4'}), 154.5 (C₂), 145.6 (C₈ₐ), 141.2 (C₈), 140.0 (C₄), 138.3 (C_{4"}), 136.5 (C_{1"}), 130.7 (C_{2"}C_{6"}), 130.5 (C_{1'}), 130.4 (C₇), 128.6 (C_{2'}C_{6'}), 127.5 (C_{3"}C_{5"}), 127.0 (C₆), 124.6 (C₅), 123.4 (C₄ₐ), 117.8 (C₃), 114.3 (C_{3'}C_{5'}), 55.3 (C₇), 34.6 (C_{7"}), 33.1 (C_{8"}), 21.8 (C_{9"}), 13.8 (C_{10"}).

### Example 44: 8-(4-(tert-butyl)phenyl)-2-(4-methoxyphenyl)quinoline-4-carboxylic acid

The product was prepared according to the methods described above and obtained as a yellow solid with a yield of 74%.
m.p.: 207.2 °C
¹H NMR (300 MHz, DMSO-d₆) δ 8.55(dd, *J* = 8.4, 1.4 Hz, 1H, H₅), 8.14 (m, 2H, H_{2"}H_{6"}), 8.11 (s, 1H, H₃), 7.73 (m, 3H, H₇H_{2'}H_{6'}), 7.59 (m, 1H, H₆), 7.54 (m, 2H, H_{3"}H_{5"}), 7.04 (m, 2H, H_{3'}H_{5'}), 3.82 (s, 3H, H_{7'}), 1.38 (s, 9H, H_{8"}).
¹³C NMR (75 MHz, DMSO-d₆) δ 168.9 (-COO), 160.5 (C_{4'}), 154.5 (C₂), 149.3 (C₈ₐ), 145.4 (C_{4"}), 139.3 (C₈), 139.7 (C₄), 131.3 (C_{1"}), 130.5 (C_{1'}), 130.5 (C_{2"}C_{6"}), 129.8 (C₇), 128.5 (C_{2'}C_{6'}), 126.1 (C₆), 125.7 (C₅), 124.3 (C_{3"}C_{5"}), 124.1 (C₄ₐ), 116.0 (C₃), 114.2 (C_{3'}C_{5'}), 55.3 (C₇), 34.3 (C_{7"}), 31.2 (C_{8"}).
HPLC (Sunfire C18, 80-95% of Acetonitrile in water, 10 min): t_{R} = 6.56min.
LC-MS (m/z): 412.3 ([M+H]⁺).

### Example 45: 8-(4-acetylphenyl)-2-(4-methoxyphenyl)quinoline-4-carboxylic acid

The product was prepared according to the methods described above and obtained as a yellow solid with a yield of 92%.
m.p.: 264.6 °C
¹H NMR (300 MHz, DMSO-d₆) δ 8.63 (dd, *J* = 8.5, 1.5 Hz, 1H, H₅), 8.45 (s, 1H, H₃), 8.12 (m, 4H, H_{2"}H_{6"}H_{3"}H_{5"}), 7.89 (m, 3H, H₇H_{2'}H_{6'}), 7.75 (dd, *J* = 8.5, 7.2 Hz, 1H, H₆), 7.07 (m, 2H, H_{3'}H_{5'}), 3.82 (s, 3H, H_{7'}), 2.67 (s, 3H, H_{8"}).
¹³C NMR (75 MHz, DMSO-d₆) δ 197.8 (C_{7"}), 167.8 (-COO), 160.9 (C_{4'}), 154.9 (C₂), 145.4 (C₈ₐ), 144.1 (C₈), 138.9 (C_{1"}), 138.3 (C₄), 135.4 (C_{4"}), 131.1 (C_{2"}C_{6"}), 130.9 (C_{1'}), 130.3 (C₇), 128.7 (C_{2'}C_{6'}), 127.5 (C_{3"}C_{5"}), 127.0 (C₆), 125.7 (C₅), 123.4 (C₄ₐ), 118.2 (C₃), 114.4 (C_{3'}C_{5'}), 55.3 (C₇), 26.8 (C_{8"}).
HPLC (Sunfire C18, 70-95% of Acetonitrile in water, 10 min): t_{R} = 1.63 min.
LC-MS (m/z): 398.3 ([M+H]⁺).

### Example 46: 8-(3,4-dichlorophenyl)-2-(4-methoxyphenyl)quinoline-4-carboxylic acid

The product was prepared according to the methods described above and obtained as a yellow solid with a yield of 60%.
m.p.: 292.5 °C
¹H NMR (300 MHz, DMSO-d₆) δ 8.61 (dd, *J* = 8.5, 1.4 Hz, 1H, H₅), 8.37 (s, 1H, H₃), 8.15 (m, 2H, H_{2'}H_{6'}), 8.06 (d, *J* = 1.8 Hz, 1H, H_{2"}), 7.89 (dd, *J* = 7.2, 1.5 Hz, 1H, H₇), 7.72 (m, 3H, H₆H_{5"}H_{6"}), 7.07 (m, 2H, H_{3'}H_{5'}), 3.83 (s, 3H, H_{7'}).
¹³C NMR (75 MHz, DMSO-d₆) δ 168.1 (-COO), 160.9 (C_{4'}), 154.9 (C₂), 145.0 (C₈ₐ), 139.7 (C₈), 136.9 (C₄), 132.7 (C_{1'}), 131.0 (C₇), 130.5 (C_{3"}), 130.5 (C_{1"}), 130.4 (C_{4"}), 130.3 (C_{2"}), 129.8 (C_{6"}), 129.7 (C_{5"}), 128.6 (C_{2'}C_{6'}), 126.6 (C₆), 126.3 (C₅), 123.6 (C₄ₐ), 117.6 (C₃), 114.3 (C_{3'}C_{5'}), 55.3 (C_{7'}).
HPLC (Sunfire C18, 60-95% of Acetonitrile in water, 10 min): t_{R} = 6.02 min.
LC-MS (m/z): 424.2 ([M]⁺).

### Example 47: 2-(4-methoxyphenyl)-8-(3-(trifluoromethoxy)phenyl)quinoline-4-carboxylic acid

The product was prepared according to the methods described above and obtained as a yellow solid with a yield of 75%.
m.p.: 166.4 °C
¹H NMR (300 MHz, DMSO-d₆) δ 8.61 (dd, *J* = 8.5, 1.5 Hz, 1H, H₅), 8.28 (s, 1H, H₃), 8.13 (m, 2H, H_{2'}H_{6'}), 7.83 (dd, *J* = 7.2, 1.5 Hz, 1H, H₇), 7.76 (m, 2H, H_{2"}H_{6"}), 7.66 (m, 2H, H₆H_{5"}), 7.44 (ddt, *J* = 8.2, 2.4, 1.1 Hz, 1H, H_{4"}), 7.02 (m, 2H, H_{3'}H_{5'}), 3.82 (s, 3H, H_{7'}).
¹³C NMR (75 MHz, DMSO-d₆) δ 168.5 (-COO), 160.7 (C_{4'}), 154.8 (C₂), 147.8 (C₈ₐ), 145.1 (C₈), 143.2 (C_{3"} (c)), 141.7 (C₄), 137.8 (C_{1'}), 130.7 (C_{1"}), 130.5 (C₇), 129.7 (C_{5"}), 129.6 (C₆), 128.5 (C_{2'}C_{6'}), 126.6 (C₈), 126.1 (C_{6"}), 123.9 (C_{2"}), 123.5 (C₄ₐ), 121.9 (C_{7"}), 119.6 (C_{4"}), 116.8 (C₃), 114.1 (C_{3'}C_{5'}), 55.5 (C_{7'}).
HPLC (Sunfire C18, 60-95% of Acetonitrile in water, 10 min): t_{R} = 5.32 min.
LC-MS (m/z): 440.2 ([M+H]⁺).

### Example 48: 2-(4-methoxyphenyl)-8-(4-propoxyphenyl)quinoline-4-carboxylic acid

The product was prepared according to the methods described above and obtained as a yellow solid with a yield of 92%.
m.p.: 211.7 °C
¹H NMR (300 MHz, DMSO-d₆) δ 8.51 (dd, *J* = 8.4, 1.5 Hz, 1H, H₅), 8.35 (s, 1H, H₃), 8.16 (m, 2H, H_{2"}H_{6"}), 7.77 (dd, *J* = 7.2, 1.5 Hz, 1H, H₇), 7.66 (m, 3H, H₆H_{2'}H_{6'}), 7.07 (m, 4H, H_{3'}H_{5'}H_{3"}H_{5"}), 4.02 (t, *J* = 6.5 Hz, 2H, H_{7"}), 3.82 (s, 3H, H₇), 1.79 (h, *J* = 7.1 Hz, 2H, H_{8"}), 1.03 (t, *J* = 7.4 Hz, 3H, H_{9"}).
¹³C NMR (75 MHz, DMSO-d₆) δ 168.3 (-COO), 160.7 (C_{4'}), 158.0 (C_{4"}), 154.3 (C₂), 145.5 (C₈ₐ), 140.1 (C₈), 139.5 (C₄), 131.9 (C_{2"}C_{6"}), 131.4 (C_{1"}), 130.6 (C_{1'}), 130.0 (C₇), 128.5 (C_{2'}C_{6'}), 126.7 (C₆), 124.6 (C₈), 123.6 (C₄ₐ), 117.2 (C₃), 114.3 (C_{3"}C_{5"}), 113.6 (C_{3'}C_{5'}), 68.9 (C_{7"}), 55.3 (C_{1'}), 22.1 (C_{8"}), 10.4 (C_{9"}).
HPLC (Sunfire C18, 60-95% of Acetonitrile in water, 10 min): t_{R} = 4.74 min.
LC-MS (m/z): 414.4 ([M+H]⁺).

### Example 49: 2-(4-methoxyphenyl)-8-(3,4,5-trimethoxyphenyl)quinoline-4-carboxylic acid

The product was prepared according to the methods described above and obtained as a yellow solid with a yield of 94%.
m.p.: 191.5 °C
¹H NMR (300 MHz, DMSO-d₆) δ 8.56 (dd, *J* = 8.5, 1.4 Hz, 1H, H₅), 8.45 (s, 1H, H₃), 8.23 (m, 2H, H_{2'}H_{6'}), 7.90 (dd, *J* = 7.2, 1.5 Hz, 1H, H₇), 7.71 (dd, *J* = 8.5, 7.2 Hz, 1H, H₆), 7.08 (m, 4H, H₃H_{5'}H_{2"}H_{6"}), 3.85 (s, 6H, H_{7"}H_{9"}), 3.83 (s, 3H, H_{8"}), 3.78 (s, 3H, H_{7'}).
¹³C NMR (75 MHz, DMSO-d₆) δ 167.9 (-COO), 160.9 (C_{4'}), 154.4 (C₂), 152.2 (C_{3"}C_{5"}), 145.5 (C₈ₐ), 139.8 (C₈), 138.2 (C_{4"}), 137.0 (C₄), 134.7 (C_{1'}), 130.7 (C_{1"}), 130.4 (C₇), 128.6 (C_{2'}C_{6'}), 127.0 (C₆), 124.7 (C₅), 123.4 (C₄ₐ), 117.8 (C₃), 114.3 (C_{3'}C_{5'}), 108.6 (C_{2"}C_{6"}), 60.1 (C_{8"}), 55.9 (C_{7"}C_{9"}), 55.3 (C_{7'}).
HPLC (Sunfire C18, 60-95% of Acetonitrile in water, 10 min): t_{R} = 1.61 min.
LC-MS (m/z): 446.4 ([M+H]⁺).

### Example 50: 2-(4-methoxyphenyl)-8-(4-morpholinophenyl)quinoline-4-carboxylic acid

The product was prepared according to the methods described above and obtained as a yellow solid with a yield of 92%.
m.p.: 265.3 °C
¹H NMR (300 MHz, DMSO-d₆) δ 8.50 (d, *J* = 8.5 Hz, 1H, H₅), 8.41 (s, 1H, H₃), 8.19 (m, 2H, H_{2'}H_{6'}), 7.79 (dd, *J* = 7.2, 1.2 Hz, 1H, H₇), 7.67 (m, 3H, H₆H_{3"}H_{5'}), 7.09 (m, 4H, H_{3'}H_{5'}H_{2"}H_{6"}), 3.83 (s, 3H, H_{7'}), 3.79 (t, *J* = 4.9 Hz, 4H, H_{8"}H_{9"}), 3.22 (t, *J* = 4.5 Hz, 4H, H_{7"}H_{10"}).
¹³C NMR (75 MHz, DMSO-d₆) δ 168.0 (-COO), 160.8 (C_{4'}), 154.3 (C₂), 150.2 (C_{4"}), 145.6 (C₈ₐ), 139.9 (C₈), 138.4 (C₄), 131.5 (C_{2"}C_{6"}), 130.6 (C_{1"}), 129.9 (C_{1'}), 129.8 (C₇), 128.6 (C_{2'}C_{6'}), 127.1 (C₆), 124.0 (C₅), 123.5 (C₄ₐ), 117.7 (C₃), 114.3 (C_{3"}C_{5"}), 114.1 (C_{3'}C_{5'}), 66.1 (C_{8"}C_{9"}), 55.3 (C₇), 48.2 (C_{7"}C_{10"}).
HPLC (Sunfire C18, 60-95% of Acetonitrile in water, 10 min): t_{R} = 1.64 min.
LC-MS (m/z): 441.5 ([M+H]⁺).

### Example 51: 6-(3-Allylamino-phenyl)-2-(4'-butyl-biphenyl-4-yl)-quinoline-4-carboxylic acid allyl ester

The product was prepared according to the method described above and obtained as a yellow oil with a yield of 40%.
¹H NMR (300 MHz, DMSO-d₆) δ 8.98 (m, 1H, H₅), 8.43 (d, *J* = 9.6 Hz, 1H, H₈), 8.32 (m, 3H, H₃H_{2'}H_{6'}), 8.07 (m, 1H, H₇), 7.82 (d, *J* = 8.2 Hz, 1H, H_{3'}H_{5'}), 7.66 (m, 3H, H_{8'}H_{12'}H_{6"}), 7.55 (d, *J* = 8.3 Hz, 1H, H_{9'}H_{11"}), 7.48 (t, *J* = 7.8 Hz, 1H, H_{5"}), 7.32 (m, 2H, H_{2"}H_{4"}), 6.15 (m, 1H, O-CH₂-CH=CH₂), 5.97 (m, 1H, NH-CH₂-CH=CH₂), 5.55 (m, 1H, O-CH₂-CH=CH₂), 5.43 (m, 1H, NH-CH₂-CH=CH₂), 5.26 (m, 1H, O-CH₂-CH=CH₂), 5.20 (m, 1H, O-CH₂), 5.04 (m, 1H, NH-CH₂-CH=CH₂), 4.31 (m, 1H, NH-CH₂), 2.10 (t, *J* = 7.7 Hz, 2H, H_{13'}), 1.72 (p, *J* = 7.6 Hz, 2H, H_{14'}), 1.43 (h, *J* = 7.5 Hz, 2H, H_{15'}), 0.99 (t, *J* = 7.3 Hz, 2H, H_{16'}).
¹³C NMR (75 MHz, DMSO-d₆) δ 167.2 (-COO), 156.9 (C₂), 154.9 (C_{10'}), 151.6 (C_{3"}), 144.2 (C₈ₐ), 143.0 (C₄), 138.5 (C_{4'}), 137.1 (C₆), 136.9 (C_{1"}), 136.3 (C_{1'}), 135.0 (C_{7'}), 132.1 (NH-CH₂-CH=CH₂), 131.0 (O-CH₂-CH=CH₂), 130.0 (C₈), 129.6 (C₅), 128.3 (C_{8'}C_{12'}), 128.0 (C_{2'}C_{6'}), 127.3 (C_{3'}C_{5'}), 126.3 (C_{9'}C_{11'}), 126.3 (C₇), 125.7 (C_{5"}), 123.9 (C₄ₐ), 123.8 (C₃), 123.8 (C_{6"}), 121.2 (C_{4"}), 121.0 (C_{2"}), 119.8 (O-CH₂-CH=CH₂), 117.1 (NH-CH₂-CH=CH₂), 62.4 (O-CH₂), 53.5 (NH-CH₂), 34.5 (C_{13'}), 33.6 (C_{14'}), 21.9 (C_{15'}), 14.0 (C_{16'}).
LC-MS (m/z): 553.5 ([M+H]⁺).

### Example 52: 8-(4-Butyl-phenyl)-2-(4-methoxy-phenyl)-quinoline-4-carboxylic acid

The product was prepared according to the methods described above and obtained as a yellow solid with a yield of 98%.
m.p.: 184.2 °C
¹H NMR (300 MHz, DMSO-d₆) δ 8.55 (m, 1H, H₅), 8.42 (s, 1H, H₃), 8.16 (m, 2H, H_{2"}H_{6"}), 7.82 (m, 1H, H₇), 7.69 (m, 3H, H₆H_{2'}H_{6'}), 7.33 (m, 2H, H_{3"}H_{5"}), 7.05 (m, 2H, H_{3'}H_{5'}), 3.82 (s, 3H, H_{7'}), 2.68 (t, *J* = 7.9 Hz, 2H, H_{7"}), 1.64 (m, 2H, H_{8"}), 1.38 (m, 2H, H_{9"}), 0.94 (m, 3H, H_{10"}).
¹³C NMR (75 MHz, DMSO-d₆) δ 168.0 (-COO), 160.8 (C_{4'}), 154.5 (C₂), 145.6 (C₈ₐ), 141.2 (C₈), 140.0 (C₄), 138.3 (C_{4"}), 136.5 (C_{1"}), 130.7 (C_{2"}C_{6"}), 130.5 (C_{1'}), 130.4 (C₇), 128.6 (C_{2'}C_{6'}), 127.5 (C_{3"}C_{5"}), 127.0 (C₆), 124.6 (C₅), 123.4 (C₄ₐ), 117.8 (C₃), 114.3 (C_{3'}C_{5'}), 55.3 (C_{7'}), 34.6 (C_{7"}), 33.1 (C_{8"}), 21.8 (C_{9"}), 13.8 (C_{10"}).
HPLC (Sunfire C18, 80-95% of Acetonitrile in water, 10 min): t_{R} = 7.52 min.
LC-MS (m/z): 412.4 ([M+H]⁺).

### Example 53: 6-[3-(Allyl-tert-butoxycarbonyl-amino)-phenyl]-2-(4'-tert-butyl-biphenyl-4-yl)-quinoline-4-carboxylic acid allyl ester

The product was prepared according to the methods described above and obtained as a yellow oil with a yield of 32%.
¹H NMR (300 MHz, DMSO-d₆) δ 9.00 (m, 1H, H₅), 8.49 (d, *J* = 9.6 Hz, 1H, H₈), 8.30 (m, 3H, H₃H_{2'}H_{6'}), 8.03 (m, 1H, H₇), 7.80 (d, *J* = 8.2 Hz, 1H, H_{3'}H_{5'}), 7.63 (m, 3H, H_{8'}H_{12'}H_{6"}), 7.52 (d, *J* = 8.3 Hz, 1H, H_{9'}H_{11'}), 7.45 (t, *J* = 7.8 Hz, 1H, H_{5"}), 7.33 (m, 2H, H_{2"}H_{4"}), 6.15 (m, 1H,O-CH₂-CH=CH₂), 5.99 (m, 1H, NH-CH₂-CH=CH₂), 5.52 (m, 1H,O-CH₂-CH=CH₂), 5.40 (m, 1H, NH-CH₂-CH=CH₂), 5.20 (m, 1H,O-CH₂), 5.26 (m, 1H,O-CH₂-CH=CH₂), 5.01 (m, 1H, NH-CH₂-CH=CH₂), 4.30 (m, 1H, NH-CH₂), 1.39 (s, 9H, H_{*t*Bu}).
¹³C NMR (75 MHz, DMSO-d₆) δ 166.8 (-COO), 156.7 (C₂), 154.9 (C_{10'}), 151.3 (C_{3"}), 143.9 (C₈ₐ), 142.8 (C₄), 138.0 (C_{4'}), 137.8 (C₆), 136.4 (C_{1"}), 136.0 (C_{1'}), 134.8 (C_{7'}), 132.1 (NH-CH₂-CH=CH₂), 131.1 (O-CH₂-CH=CH₂), 129.9 (C₈), 129.6 (C₅), 128.3 (C_{8'}C_{12'}), 128.0 (C_{2'}C_{6'}), 127.3 (C_{3'}C_{5'}), 126.3 (C_{9'}C_{11'}), 126.0 (C₇), 125.4 (C_{5"}), 124.7 (C₄ₐ), 123.8 (C₃), 123.8 (C_{6"}), 121.0 (C_{4"}), 121.0 (C_{2"}), 119.8 (O-CH₂-CH=CH₂), 117.1 (NH-CH₂-CH=CH₂), 62.4 (O-CH₂), 53.5 (NH-CH₂). 35.0 (C_{13'}), 28.8 (C_{*t*Bu}).
HPLC (Sunfire C18, 90-95% of Acetonitrile in water, 10 min): t_{R} = 7.92 min.
LC-MS (m/z): 653.5 ([M+H]⁺).

### Example 54: 2-(4'-Morpholin-4-yl-biphenyl-3-yl)-quinoline-4-carboxylic acid

The product was prepared according to the methods described above and obtained as a brown solid with a yield of 81%;
m.p: 110.7 °C
**¹H NMR** (400 MHz DMSO-d₆) δ 8.65 (dd, *J* = 8.5, 1.3 Hz, 1H, H₅), 8.55 (s, 1H, H₃), 8.46 (t, *J* = 1.8 Hz, 1H, H_{2'}), 8.19 (t, *J* = 7.1 Hz, 2H, H₈H_{6'}), 7.86 (m, 1H, H₇), 7.73 (m, 2H, H₆H_{4'}), 7.67 (m, 2H, H_{2"}H_{6"}), 7.60 (t, *J* = 7.7 Hz, 1H, H_{5'}), 7.04 (m, 2H, H_{3"}H_{5"}), 3.75 (t, *J* = 4.6 Hz, 4H, H_{8"}H_{9"}), 3.15 (t, *J* = 4.6 Hz, 4H, H_{7"}H_{10"}).
**¹³C NMR** (100 MHz, DMSO-d₆) δ 167.8 (COO-), 156.0 (C₂), 150.7 (C_{4"}), 148.4 (C₈ₐ), 140.8 (C₄), 138.6 (C_{3'}), 137.8 (C_{1'}), 130.3 (C_{1"}), 130.2 (C₄ₐ), 129.9 (C_{2'}), 129.6 (C_{5'}), 127.8 (C₇), 127.5 (C₈), 127.4 (C_{2"}C_{6"}), 125.4 (C₆), 125.4 (C₅), 124.6 (C_{6'}), 123.5 (C_{4'}), 119.4 (C₃), 115.3 (C_{3"}C_{5"}), 66.1 (C_{7"}C_{10"}), 48.2 (C_{8"}C_{9"}).
**HPLC** (Sunfire C18, 50-95% of Acetonitrile in water, 10 min): t_{R} = 7.00 min.
**LC-MS (m/z):** 354.3 ([M+H]⁺).

### Example 55: 2-(3',4',5'-trimethoxy-[1,1'-biphenyl]-3-yl)quinoline-4-carboxylic acid

The product was prepared according to the methods described above and obtained as a yellow solid with a yield of 96%;
m.p: 206.3 °C
**¹H NMR** (400 MHz DMSO-d₆) δ 8.64 (dd, *J* = 8.6, 1.4 Hz, 1H, H₅), 8.56 (s, 1H, H₃), 8.48 (t, *J* = 1.9 Hz, 1H, H_{2'}), 8.25 (m, 2H, H₈H_{6'}), 7.88 (m, 1H, H₇), 7.84 (m, 1H, H₆), 7.74 (m, 1H, H_{4'}), 7.66 (t, *J* = 7.7 Hz, 1H, H_{5'}), 7.03 (s, 2H, H_{2"}H_{6"}), 3.90 (s, 6H, H_{7"}H_{9"}), 3.72 (s, 3H, H_{8"}).
**¹³C NMR** (100 MHz, DMSO-d₆) δ 167.6 (COO-), 155.9 (C₂), 153.4 (C_{3"}C_{5"}), 147.9 (C₈ₐ), 141.4 (C₄), 138.4 (C_{4"}), 138.2 (C_{1'}), 137.4 (C_{3'}), 135.9 (C_{1"}), 130.6 (C₄ₐ), 129.6 (C_{2'}), 129.5 (C_{5'}), 128.9 (C₇), 128.1 (C₈), 126.5 (C_{6'}), 125.9 (C₆), 125.5 (C₅), 123.6 (C_{4'}), 119.6 (C₃), 104.6 (C_{2"}C_{6"}), 60.4 (C_{8"}), 56.0 (C_{7"}C_{9"}).
**HPLC** (Sunfire C18, 50-95% of Acetonitrile in water, 10 min): t_{R} = 7.00 min.
**LC-MS (m/z):** 354.3 ([M+H]⁺).

### Example 56: 2-(3',4'-dichloro-[1,1'-biphenyl]-3-yl)quinoline-4-carboxylic acid

The product was prepared according to the methods described above and obtained as a white solid with a yield of 57%;
m.p: 248.2 °C
**¹H NMR** (400 MHz DMSO-d₆) δ 8.61 (dd, *J* = 8.6, 1.3 Hz, 1H, H₅), 8.58 (s, 1H, H₃), 8.54 (t, *J* = 1.8 Hz, 1H, H_{2'}), 8.35 (dt, *J* = 7.9, 1.3 Hz, 1H, H_{6'}), 8.20 (dd, *J* = 8.5, 1.1 Hz, 1H, H₈), 8.12 (d, *J* = 2.1 Hz, 1H, H_{2"}), 7.87 (m, 2H, H₆H_{4'}), 7.83 (dd, *J* = 8.4, 2.1 Hz, 1H, H_{6"}), 7.76 (d, *J* = 8.4 Hz, 1H, H_{5"}), 7.68 (m, 2H, H₇H_{5'}).
**¹³C NMR** (100 MHz, DMSO-d₆) δ 167.8 (COO-), 155.6 (C₂), 148.3 (C₈ₐ), 140.6 (C₄), 138.9 (C_{1'}), 138.5 (C_{3'}), 138.5 (C_{1"}), 131.8 (C_{3"}), 131.0 (C_{2'}), 130.5 (C_{4"}), 130.3 (C₄ₐ), 129.8 (C_{2"}), 129.8 (C_{5"}), 128.9 (C_{5'}), 128.5 (C₇), 127.9 (C_{6"}), 127.3 (C₈), 127.3 (C₆), 125.7 (C_{6'}), 125.5 (C₅), 123.5 (C_{4'}), 119.2 (C₃).
**HPLC** (Sunfire C18, 50-95% of Acetonitrile in water, 10 min): t_{R} = 7.00 min.
**LC-MS (m/z):** 354.3 ([M+H]⁺).

### Example 57: 2-(4'-propoxy-[1,1'-biphenyl]-3-yl)quinoline-4-carboxylic acid

The product was prepared according to the methods described above and obtained as a yellow solid with a yield of 70%;
m.p: 201.6 °C
**¹H NMR** (400 MHz DMSO-d₆) δ 8.64 (dd, *J* = 8.6, 1.3 Hz, 1H, H₅), 8.55 (s, 1H, H₃), 8.47 (t, *J* = 1.8 Hz, 1H, H_{2'}), 8.21 (m, 2H, H₈H_{6'}), 7.86 (m, 1H, H₇), 7.76 (m, 1H, H₆), 7.72 (m, 3H, H_{4'}H_{2"}H_{6"}), 7.63 (t, *J=* 7.7 Hz, 1H, H_{5'}), 7.06 (m, 2H, H_{3"}H_{5"}), 3.98 (t, *J* = 6.5 Hz, 2H, H_{7"}), 1.75 (h, *J* = 7.1 Hz, 2H, H_{8"}), 0.99 (t, *J* = 7.4 Hz, 3H, H_{9"}).
**¹³C NMR** (100 MHz, DMSO-d₆) δ 167.8 (COO-), 158.6 (C_{4"}), 155.9 (C₂), 148.4 (C₈ₐ), 140.7 (C₄), 138.6 (C_{3'}), 137.8 (C_{1'}), 132.1 (C_{1"}), 130.3 (C₄ₐ), 129.9 (C_{2'}), 129.6 (C_{5'}), 128.1 (C_{3"}C_{5"}), 127.9 (C₇), 127.9 (C₈), 125.7 (C₆), 125.4 (C₅), 125.0 (C_{6'}), 123.5 (C_{4'}), 119.4 (C₃), 115.0 (C_{2"}C_{6"}), 69.1 (C_{7"}), 22.1 (C_{8"}), 10.5 (C_{9"}).
**HPLC** (Sunfire C18, 50-95% of Acetonitrile in water, 10 min): t_{R} = 7.00 min.
**LC-MS (m/z):** 354.3 ([M+H]⁺).

### Example 58: 6-(4-morpholinophenyl)-2-phenylquinoline-4-carboxylic acid

The product was prepared according to the methods described above and obtained as a orange solid with a yield of 76%;
**¹H NMR** (400 MHz, DMSO-d₆) δ 8.87 (d, *J* = 1.9 Hz, 1H, H₅), 8.46 (s, 1H, H₃), 8.30 (m, 2H, H_{2'}H_{6'}), 8.19 (d, *J* = 8.8 Hz, 1H, H₈), 8.14 (dd, *J* = 8.9, 2.0 Hz, 1H, H₇), 7.71 (m, 2H, H_{3"}H_{5"}), 7.57 (m, 3H, H_{4'}H_{3'}H_{5'}), 7.12 (m, 2H, H_{2"}H_{6"}), 3.77 (t, *J* = 4.8 Hz, 4H, H_{8"}H_{9"}), 3.21 (t, *J* = 4.7 Hz, 4H, H_{7"}H_{10"}).
**¹³C NMR** (100 MHz, DMSO-d₆) δ 167.6 (COO-), 155.1 (C₂), 150.9 (C₄), 147.5 (C₈ₐ), 138.9 (C₆), 138.0 (C_{1"}), 130.2 (C_{4"}), 130.2 (C_{1'}), 129.9 (C₈), 129.6 (C₇), 129.0 (C_{2"}C_{6"}), 129.0 (C_{4'}), 127.7 (C_{2'}C_{6'}), 127.1 (C_{3"}C_{5"}), 124.0 (C₄ₐ), 121.2 (C₅), 119.0 (C₃), 115.3 (C_{3'}C_{5'}), 66.1 (C_{8"}C_{9"}), 48.0 (C_{7"}C_{10"}).
**HPLC** (Sunfire C18, 60-95% of Acetonitrile in water, 10 min): t_{R} = 1.59 min.
**LC-MS (m/z):** 441.5 ([M+H]⁺).

### Example 59: 2-phenyl-6-(4-propoxyphenyl)quinoline-4-carboxylic acid

The product was prepared according to the methods described above and obtained as a yellow solid with a yield of 82%;
m.p: 216.5 °C
**¹H NMR** (400 MHz, DMSO-d₆) δ 8.87 (d, *J* = 2.0 Hz, 1H, H₅), 8.49 (s, 1H, H₃), 8.30 (m, 2H, H_{2'}H_{6'}), 8.20 (d, *J* = 8.8 Hz, 1H, H₈), 8.13 (dd, *J* = 8.8, 2.1 Hz, 1H, H₇), 7.73 (m, 2H, H_{3"}H_{5"}), 7.57 (m, 3H, H_{4'}H_{3'}H_{5'}), 7.10 (m, 2H, H_{2"}H_{6"}), 4.00 (t, *J=* 6.5 Hz, 2H, H_{7"}), 1.76 (h, *J* = 7.1 Hz, 2H, H_{8"}), 1.00 (t, *J* = 7.4 Hz, 3H, H_{9"}).
**¹³C NMR** (100 MHz, DMSO-d₆) δ 167.6 (COO-), 158.9 (C₂), 155.4 (C₄), 147.7 (C₈ₐ), 138.9 (C₆), 137.9 (C_{1"}), 137.2 (C_{4"}), 131.6 (C_{1'}), 130.3 (C₈), 130.0 (C₇), 129.0 (C_{4'}), 129.0 (C_{2'}C_{6'}), 128.3 (C_{2"}C_{6"}), 127.2 (C_{3"}C_{5"}), 123.9 (C₄ₐ), 121.8 (C₅), 119.7 (C₃), 115.2 (C_{3'}C_{5'}), 69.1 (C_{7"}), 22.1 (C_{8"}), 10.5 (C_{9"}).
**HPLC** (Sunfire C18, 60-95% of Acetonitrile in water, 10 min): t_{R} = 4.15 min.
**LC-MS (m/z):** 414.4 ([M+H]⁺).

### Example 60: 2-phenyl-6-(3,4,5-trimethoxyphenyl)quinoline-4-carboxylic acid

The product was prepared according to the methods described above and obtained as a orange solid with a yield of 85%;
m.p: 233.6 °C
**¹H NMR** (400 MHz, DMSO-d₆) δ 8.88 (d, *J* = 1.4 Hz, 1H, H₅), 8.50 (s, 1H, H₃), 8.31 (m, 2H, H_{2'}H_{6'}), 8.23 (d, *J* = 8.2 Hz, 1H, H₈), 8.20 (dd, *J* = 8.8, 2.0 Hz, 1H, H₇), 7.58 (m, 3H, H_{4'}H_{3'}H_{5'}), 7.05 (s, 2H, H_{2"}H_{6"}), 3.90 (s, 6H, H_{7"}H_{9"}), 3.73 (s, 3H, H_{8"}).
**¹³C NMR** (100 MHz, DMSO-d₆) δ 167.6 (COO-), 155.7 (C₂), 153.4 (C_{3"}C_{5"}), 147.8 (C₈ₐ), 139.5 (C₆), 137.8 (C_{1'}), 137.7 (C_{4"}), 137.6 (C₄), 135.4 (C_{1"}), 130.2 (C₈), 130.1 (C₇), 129.8 (C_{4'}), 129.1 (C_{3'}C_{5'}), 127.2 (C_{2'}C_{6'}), 123.7 (C₅), 122.9 (C₄ₐ), 119.7 (C₃), 104.9 (C_{2"}C_{6"}), 60.2 (C_{8"}), 56.1 (C_{7"}C_{9"}).
**HPLC** (Sunfire C18, 60-95% of Acetonitrile in water, 10 min): t_{R} = 1.67 min.
**LC-MS (m/z):** 446.3 ([M+H]⁺).

### Materials and methods

### Antiviral activity against SARS-CoV-2

Vero-E6 cells (ATCC No. CRL-1586) were seeded onto 96-well plates (2.5 X10⁴ cells/well). Candidate compounds were diluted from stock solutions in complete media [(DMEM supplemented with 10 mM HEPES, 1X non-essential amino acids (Gibco), 100 U/mL penicillin-streptomycin (Gibco) and 2 % Fetal Bovine Serum (heat-inactivated at 56 °C for 30 min)) to achieve the desired concentration. A wide range of dilutions from 50µM to 780nM were tested initially. SARS-CoV-2 stock strain NL/2020 (kindly provided by Dr. R. Molenkamp, Erasmus University Medical Center Rotterdam)) was diluted to achieve a multiplicity of infection (MOI) of 0.001. Virus-compound mixture was used to inoculate the Vero-E6 monolayer. Cultures were maintained at 37°C in a 5% CO₂ incubator for 72 hours in the BSL3 facility. Cells were fixed using a 4% formaldehyde-PBS solution to finalize the experiment and inactivate the virus. Virus-induced cell death was revealed by staining with a 0.1% crystal violet solution in water-methanol for 30-60 minutes, time after which, plates were extensively washed with water and dried. Infected as well as uninfected cell wells were used as controls. Additionally, remdesivir was used as reference compound. Using crystal violet staining, we determined the dilutions of extract capable of protecting the cell monolayer from virus-induced cell death.

The antiviral activity was initially estimated by the maximum and minimum protective concentrations (PCmax and PCmin), i.e. maximum and minimum compound concentrations capable of protecting a Vero-E6 cell monolayer from virus induced cell death.

**Table 1: Antiviral activity on SARS-CoV-2**

| Example | PC Max | PC Min |
|---|---|---|
| Remdesevir | 50 | 0.78 |
| 3 | 50 | 50 |
| 4 | 50 | 50 |
| 12 | 50 | 50 |
| 14 | 50 | 50 |
| 16 | 50 | 50 |
| 19 | 50 | 50 |
| 20 | 50 | 12.50 |
| 21 | 50 | 3.125 |
| 23 | 50 | 50 |
| 24 | 50 | 50 |
| 27 | 50 | 25 |
| 28 | 50 | 25 |
| 33 | 50 | 50 |
| 34 | 50 | 50 |
| 42 | 50 | 50 |
| 44 | 50 | 12.50 |
| 46 | 50 | 50 |
| 47 | 50 | 50 |
| 48 | 50 | 50 |

Given that protection of the cell monolayer is an indirect evaluation of the compound antiviral activity, and to confirm that protection of the monolayer was indeed due to the ability of the compounds to interfere with virus replication, we determined the relative viral RNA contents in the presence/absence of the compounds to directly infer the antiviral activity of the candidates. Thus, we extracted total cellular RNA from 2.5 X10⁴ cells treated with 200µl of cell culture media supplemented with compounds to achieve a final 15µM concentration of each of the compounds or with the corresponding volume of the vehicle to determine the relative viral load found in mock-infected as well as infected cells in the presence/absence of the extracts by real time qPCR. We included in the analysis cells infected in the absence of vehicle (media) as well as mock-infected cells as positive and negative controls respectively. The cell morphology was not affected by the presence of the controls (not shown). The results are expressed as a percentage of the quantity of RNA in comparison with the quantity detected when the cells where only treated with the vehicle (DMSO).

**Table 2. qPCR results for the compounds of examples 21, 44, 20.**

| Compound | % RNA relative to DMSO |
|---|---|
| DMSO | 10² |
| 20 | 10⁻³ |
| 21 | 10⁻⁵ |
| 44 | 10⁻² |

The qPCR results indicate that the compounds of examples 21, 44 and 20 are particularly potent antivirals at 15µM, reaching virtually undetectable levels as compared with the uninfected controls.

### Activity on human coronavirus 229E

Huh7-Lunet#3 cells (kindly provided by Dr. Thommas Pietschmann; Twincore-Hannover) were maintained subconfluent in complete media [(DMEM supplemented with 10 mM HEPES, 1X non-essential amino acids (Gibco), 100 U/ mL penicillin-streptomycin (Gibco) and 10 % Fetal Bovine Serum (FBS; heat-inactivated at 56 °C for 30 min)].

Huh7-Lunet#3 cells were seeded onto 96-well plates (1x10⁴ cells/well). The day after, compound stock solutions (10 mM in DMSO) were diluted into complete cell culture media to achieve a final concentration of 20 µM. On the other hand, hCoV-229E-GFP virus (Human coronavirus 229E expressing Green Fluorescent Protein) stock (kindly provided by Dr. Volker Thiel; University of Bern) was diluted in complete media to achieve a final concentration of 3x10³ focus forming units (FFU)/ml. One hundred microliters (100 µl) of the virus dilution were mixed 1:1 with 100 µl of the compound dilution to achieve final compound concentrations of 10 µM and 150 infectious units (FFU) per well in a 96 well plate. One hundred µl of the mixture was applied onto the Huh7-Lunet #3 cell monolayer in biological replicates and cells were cultured for 72 hours at 33°C in a 5% CO₂ incubator. Cells were fixed in a 4% formaldehyde solution in PBS for 10 minutes at room temperature, washed twice with PBS and individual well fluorescence was measured in a SpectraMax iD3 fluorescence plate reader (Molecular Devices). Background subtraction was performed using non-infected wells and signal was normalized to the average fluorescence found in vehicle (DMSO)-treated virus-infected wells. Once infection efficiency had been determined, plates were stained with a 0.1% crystal violet solution in water-methanol for 30-60 minutes. Then, plates were extensively washed with water and dried before 1% SDS solution in water was added to solubilize crystal violet. Absorbance was measured at 570 nm and background was subtracted from blank wells. Relative well biomass was estimated by calculating the absorbance in each well relative to the average observed in infected cells treated with DMSO.

Compound of example **4** (10µM) reduced by 74% hCoV-229E-GFP propagation in the absence of measurable cytotoxicity, given that well biomass was comparable (107%) to that found in the controls, suggesting that it also displays antiviral activity against hCoV-229E-GFP.

## Claims

1. A compound of formula (I) 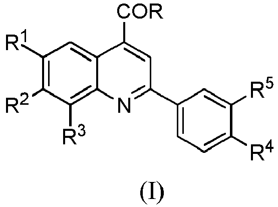 for use in the treatment and/or prevention of an infection by a virus of the family *Coronaviridae,* wherein
• • R is a group selected from hydroxyl, OR' and NR'R" wherein R' and R" independently represent a group selected from allyl, benzyl, C₁₋₆ alkyl and -(O-CH₂-CH₂)ₙ-OCH₃, wherein n is an integer from 1 to 25,
• R¹, R² and R³ independently represent a hydrogen atom, a phenyl or a 5-6 membered, monocyclic, heteroaromatic ring comprising 1, 2 or 3 heteroatoms selected from N, O and S, which are optionally substituted by 1, 2 or 3 groups selected from the group consisting of C₁₋₄ alkyl, C₁₋₄ alkoxy, halogen atoms, acetyl, allylamino, morpholino and trifluoromethoxy,
• R⁴ is hydrogen, methoxy, phenyl or a 5-6 membered, monocyclic, heteroaromatic ring comprising 1, 2 or 3 heteroatoms selected from N, O and S, which are optionally substituted by 1 or 2 groups selected from the group consisting of C₁₋₄ alkyl, halogen atoms, acetyl and trifluoromethoxy, and
• R⁵ is hydrogen, halogen, phenyl or a 5-6 membered, monocyclic, heteroaromatic ring comprising 1, 2 or 3 heteroatoms selected from N, O and S, which are optionally substituted by 1, 2 or 3 groups selected from the group consisting of halogen atoms, C₁₋₄ alkyl, C₁₋₄ alkoxy, acetyl, morpholino and trifluoromethoxy,
with the condition that at least one of R¹, R², R³, R⁴ and R⁵, is not hydrogen and pharmaceutically acceptable salts thereof.

2. Compound for use according to claim 1 wherein
• R is a hydroxyl, group,
• R¹, R² and R³ independently represent a hydrogen atom or a phenyl optionally substituted by 1, 2 or 3 groups selected from the group consisting of C₁₋₄ alkyl, C₁₋₄ alkoxy, halogen atoms, acetyl, morpholino and trifluoromethoxy,
• R⁴ is hydrogen, methoxy or phenyl optionally substituted by 1 or 2 groups selected from the group consisting of C₁₋₄ alkyl, halogen atoms, acetyl and trifluoromethoxy, and
• R⁵ is hydrogen, halogen or phenyl optionally substituted by 1, 2 or 3 groups selected from the group consisting of halogen atoms, C₁₋₄ alkyl, C₁₋₄ alkoxy, acetyl, morpholino and trifluoromethoxy.

3. Compound for use according to any one of claims 1 to 2 wherein at least one at least one of R¹, R², R³, R⁴ and R⁵ is a phenyl group substituted by 1 or 2 groups selected from chlorine atoms, trifluoromethoxy and C₁₋₄ alkyl.

4. Compound for use according to claim 3 wherein R¹ is a hydrogen atom and at least one at least one of R², R³, R⁴ and R⁵ is a phenyl group substituted by 1 group selected C₃₋₄ alkyl.

5. Compound for use according to claim 4 wherein R¹, R² and R⁵ are hydrogen atoms, one of R³ and R⁴ is a phenyl group substituted by 1 butyl and the other one of R³ and R⁴ is selected from hydrogen atom and methoxy.

6. Compound for use according to claim 1 wherein the compound is one of:
• 2-(4-methoxyphenyl)-6-phenylquinoline-4-carboxylic acid
• 6-(4-ethylphenyl)-2-(4-methoxyphenyl)quinoline-4-carboxylic acid
• 6-(4-butylphenyl)-2-(4-methoxyphenyl)quinoline-4-carboxylic acid
• 6-(4-(tert-butyl)phenyl)-2-(4-methoxyphenyl)quinoline-4-carboxylic acid
• 6-(4-acetylphenyl)-2-(4-methoxyphenyl)quinoline-4-carboxylic acid
• 6-(3,4-dichlorophenyl)-2-(4-methoxyphenyl)quinoline-4-carboxylic acid
• 2-(4-methoxyphenyl)-6-(3-(trifluoromethoxy)phenyl)quinoline-4-carboxylic acid
• 2-(4-methoxyphenyl)-6-(4-propoxyphenyl)quinoline-4-carboxylic acid
• 2-(4-methoxyphenyl)-6-(3,4,5-trimethoxyphenyl)quinoline-4-carboxylic acid
• 2-(4-methoxyphenyl)-6-(4-morpholinophenyl)quinoline-4-carboxylic acid
• 2,6-diphenylquinoline-4-carboxylic acid
• 6-(4-ethylphenyl)-2-phenylquinoline-4-carboxylic acid
• 6-(4-butylphenyl)-2-phenylquinoline-4-carboxylic acid
• 6-(4-(tert-butyl)phenyl)-2-phenylquinoline-4-carboxylic acid
• 6-(4-acetylphenyl)-2-phenylquinoline-4-carboxylic acid
• 6-(3,4-dichlorophenyl)-2-phenylquinoline-4-carboxylic acid
• 2-phenyl-6-(3-(trifluoromethoxy)phenyl)quinoline-4-carboxylic acid
• 2-([1,1'-biphenyl]-4-yl)quinoline-4-carboxylic acid
• 2-(4'-ethyl-[1,1'-biphenyl]-4-yl)quinoline-4-carboxylic acid
• 2-(4'-butyl-[1,1'-biphenyl]-4-yl)quinoline-4-carboxylic acid
• 2-(4'-(tert-butyl)-[1,1'-biphenyl]-4-yl)quinoline-4-carboxylic acid
• 2-(4'-acetyl-[1,1'-biphenyl]-4-yl)quinoline-4-carboxylic acid
• 2-(3',4'-dichloro-[1,1'-biphenyl]-4-yl)quinoline-4-carboxylic acid
• 2-(3'-(trifluoromethoxy)-[1,1'-biphenyl]-4-yl)quinoline-4-carboxylic acid
• 2-([1,1'-biphenyl]-3-yl)quinoline-4-carboxylic acid
• 2-(4'-ethyl-[1,1'-biphenyl]-3-yl)quinoline-4-carboxylic acid
• 2-(4'-butyl-[1,1'-biphenyl]-3-yl)quinoline-4-carboxylic acid
• 2-(4'-(tert-butyl)-[1,1'-biphenyl]-3-yl)quinoline-4-carboxylic acid
• 2-(4'-acetyl-[1,1'-biphenyl]-3-yl)quinoline-4-carboxylic acid
• 2-(3'-(trifluoromethoxy)-[1,1'-biphenyl]-3-yl)quinoline-4-carboxylic acid
• 2-(3-bromophenyl)quinoline-4-carboxylic acid
• 7-(4-ethylphenyl)-2-(4-methoxyphenyl)quinoline-4-carboxylic acid
• 7-(4-butylphenyl)-2-(4-methoxyphenyl)quinoline-4-carboxylic acid
• 7-(4-(tert-butyl)phenyl)-2-(4-methoxyphenyl)quinoline-4-carboxylic acid
• 7-(4-acetylphenyl)-2-(4-methoxyphenyl)quinoline-4-carboxylic acid
• 7-(3,4-dichlorophenyl)-2-(4-methoxyphenyl)quinoline-4-carboxylic acid
• 2-(4-methoxyphenyl)-7-(3-(trifluoromethoxy)phenyl)quinoline-4-carboxylic acid
• 2-(4-methoxyphenyl)-7-(4-propoxyphenyl)quinoline-4-carboxylic acid
• 2-(4-methoxyphenyl)-7-(3,4,5-trimethoxyphenyl)quinoline-4-carboxylic acid
• 2-(4-methoxyphenyl)-7-(4-morpholinophenyl)quinoline-4-carboxylic acid
• 2-(4-methoxyphenyl)-8-phenylquinoline-4-carboxylic acid
• 8-(4-ethylphenyl)-2-(4-methoxyphenyl)quinoline-4-carboxylic acid
• 8-(4-butylphenyl)-2-(4-methoxyphenyl)quinoline-4-carboxylic acid
• 8-(4-(tert-butyl)phenyl)-2-(4-methoxyphenyl)quinoline-4-carboxylic acid
• 8-(4-acetylphenyl)-2-(4-methoxyphenyl)quinoline-4-carboxylic acid
• 8-(3,4-dichlorophenyl)-2-(4-methoxyphenyl)quinoline-4-carboxylic acid
• 2-(4-methoxyphenyl)-8-(3-(trifluoromethoxy)phenyl)quinoline-4-carboxylic
• 2-(4-methoxyphenyl)-8-(4-propoxyphenyl)quinoline-4-carboxylic acid
• 2-(4-methoxyphenyl)-8-(3,4,5-trimethoxyphenyl)quinoline-4-carboxylic acid
• 2-(4-methoxyphenyl)-8-(4-morpholinophenyl)quinoline-4-carboxylic acid
• 6-(3 -Allylamino-phenyl)-2-(4'-butyl-biphenyl-4-yl)-quinoline-4-carboxylic acid allyl ester
• 8-(4-Butyl-phenyl)-2-(4-methoxy-phenyl)-quinoline-4-carboxylic acid
• 6-[3-(Allyl-tert-butoxycarbonyl-amino)-phenyl]-2-(4'-tert-butyl-biphenyl-4-yl)-quinoline-4-carboxylic acid allyl ester
• 2-(4'-Morpholin-4-yl-biphenyl-3-yl)-quinoline-4-carboxylic acid
• 2-(3',4',5'-trimethoxy-[1,1'-biphenyl]-3-yl)quinoline-4-carboxylic acid
• 2-(3',4'-dichloro-[1,1'-biphenyl]-3-yl)quinoline-4-carboxylic acid
• 2-(4'-propoxy-[1,1'-biphenyl]-3-yl)quinoline-4-carboxylic acid
• 6-(4-morpholinophenyl)-2-phenylquinoline-4-carboxylic acid
• 2-phenyl-6-(4-propoxyphenyl)quinoline-4-carboxylic acid
• 2-phenyl-6-(3,4,5-trimethoxyphenyl)quinoline-4-carboxylic acid
• 6-(4-ethylphenyl)-2-phenylquinoline-4-carboxylic acid
• 6-(3,4-Dichloro-phenyl)-2-phenyl-quinoline-4-carboxylic acid and pharmaceutically acceptable salts thereof.

7. A compound for use according to any one of claims 1 to 6 wherein the infection by a virus is selected from infections by respiratory syndrome-related coronaviruses selected from the species *"Severe respiratory syndrome-related coronavirus"* and *"Middle East respiratory syndrome-related coronavirus".*

8. Compound for use according any one of claims 1 to 6, wherein the respiratory syndrome-related coronavirus is selected from the group consisting of SARS-CoV-2, SARS-CoV and MERS-CoV.

9. Compound for use according to any one of the preceding claims, wherein the viral infection is by SARS-CoV-2 virus.

10. A compound of formula (I') 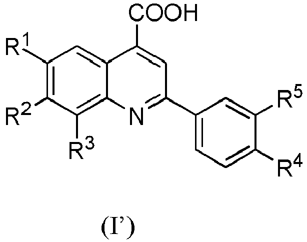 wherein
• R¹ is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
• R² is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
• R³ is hydrogen or phenyl optionally substituted by 1, 2 or 3 groups selected from the group consisting of C₁₋₄ alkyl, C₁₋₄ alkoxy, halogen atoms, acetyl, morpholino and trifluoromethoxy,
• R⁴ is hydrogen, methoxy or phenyl optionally substituted by 1 or 2 groups selected from the group consisting of C₁₋₄ alkyl, acetyl, halogen atoms and trifluoromethoxy,
• R⁵ is hydrogen, halogen or phenyl optionally substituted by 1 group selected from the group consisting of halogen atoms, C₁₋₄ alkyl, acetyl and trifluoromethoxy,
with the proviso that at least one of R¹ and R² is a phenyl group optionally substituted by 1, 2 or 3 groups selected from the group consisting of C₁₋₄ alkyl, C₁₋₄ alkoxy, halogen atoms, acetyl, morpholino and trifluoromethoxy,
and the further proviso that the compound is neither 2-(4-methoxy-phenyl)-quinoline-4-carboxylic acid nor (2,7-diphenyl-quinolin-4-carboxylic acid)
and pharmaceutically acceptable salts thereof.

11. Compound according to claim 10 wherein at least one at least one of R¹, R², R³, R⁴ and R⁵ is a phenyl group substituted by 1 or 2 groups selected from chlorine atoms, trifluoromethoxy and C₁₋₄ alkyl.

12. Compound according to claim 11 wherein R¹ is a hydrogen atom and at least one at least one of R², R³, R⁴ and R⁵ is a phenyl group substituted by 1 group selected C₃₋₄ alkyl.

13. Compound according to claim 12 wherein R¹ and R⁵ are hydrogen atoms, one of R³ and R⁴ is a phenyl group substituted by 1 butyl and the other one of R³ and R⁴ is selected from hydrogen atom and methoxy.

14. A compound according to claim 10 which is one of:
• 2-(4-methoxyphenyl)-6-phenylquinoline-4-carboxylic acid
• 6-(4-ethylphenyl)-2-(4-methoxyphenyl)quinoline-4-carboxylic acid
• 6-(4-butylphenyl)-2-(4-methoxyphenyl)quinoline-4-carboxylic acid
• 6-(4-(tert-butyl)phenyl)-2-(4-methoxyphenyl)quinoline-4-carboxylic acid
• 6-(4-acetylphenyl)-2-(4-methoxyphenyl)quinoline-4-carboxylic acid
• 6-(3,4-dichlorophenyl)-2-(4-methoxyphenyl)quinoline-4-carboxylic acid
• 2-(4-methoxyphenyl)-6-(3-(trifluoromethoxy)phenyl)quinoline-4-carboxylic acid
• 2-(4-methoxyphenyl)-6-(4-propoxyphenyl)quinoline-4-carboxylic acid
• 2-(4-methoxyphenyl)-6-(3,4,5-trimethoxyphenyl)quinoline-4-carboxylic acid
• 2-(4-methoxyphenyl)-6-(4-morpholinophenyl)quinoline-4-carboxylic acid
• 2,6-diphenylquinoline-4-carboxylic acid
• 6-(4-ethylphenyl)-2-phenylquinoline-4-carboxylic acid
• 6-(4-butylphenyl)-2-phenylquinoline-4-carboxylic acid
• 6-(4-(tert-butyl)phenyl)-2-phenylquinoline-4-carboxylic acid
• 6-(4-acetylphenyl)-2-phenylquinoline-4-carboxylic acid
• 6-(3,4-dichlorophenyl)-2-phenylquinoline-4-carboxylic acid
• 2-phenyl-6-(3-(trifluoromethoxy)phenyl)quinoline-4-carboxylic acid
• 2-(4'-butyl-[1,1'-biphenyl]-4-yl)quinoline-4-carboxylic acid
• 2-(4'-(tert-butyl)-[1,1'-biphenyl]-4-yl)quinoline-4-carboxylic acid
• 2-(4'-acetyl-[1,1'-biphenyl]-4-yl)quinoline-4-carboxylic acid
• 2-(3',4'-dichloro-[1,1'-biphenyl]-4-yl)quinoline-4-carboxylic acid
• 2-(3'-(trifluoromethoxy)-[1,1'-biphenyl]-4-yl)quinoline-4-carboxylic acid
• 2-([1,1'-biphenyl]-3-yl)quinoline-4-carboxylic acid
• 2-(4'-ethyl-[1,1'-biphenyl]-3-yl)quinoline-4-carboxylic acid
• 2-(4'-butyl-[1,1'-biphenyl]-3-yl)quinoline-4-carboxylic acid
• 2-(4'-(tert-butyl)-[1,1'-biphenyl]-3-yl)quinoline-4-carboxylic acid
• 2-(4'-acetyl-[1,1'-biphenyl]-3-yl)quinoline-4-carboxylic acid
• 2-(3'-(trifluoromethoxy)-[1,1'-biphenyl]-3-yl)quinoline-4-carboxylic acid
• 7-(4-ethylphenyl)-2-(4-methoxyphenyl)quinoline-4-carboxylic acid
• 7-(4-butylphenyl)-2-(4-methoxyphenyl)quinoline-4-carboxylic acid
• 7-(4-(tert-butyl)phenyl)-2-(4-methoxyphenyl)quinoline-4-carboxylic acid
• 7-(4-acetylphenyl)-2-(4-methoxyphenyl)quinoline-4-carboxylic acid
• 7-(3,4-dichlorophenyl)-2-(4-methoxyphenyl)quinoline-4-carboxylic acid
• 2-(4-methoxyphenyl)-7-(3-(trifluoromethoxy)phenyl)quinoline-4-carboxylic acid
• 2-(4-methoxyphenyl)-7-(4-propoxyphenyl)quinoline-4-carboxylic acid
• 2-(4-methoxyphenyl)-7-(3,4,5-trimethoxyphenyl)quinoline-4-carboxylic acid
• 2-(4-methoxyphenyl)-7-(4-morpholinophenyl)quinoline-4-carboxylic acid
• 8-(4-ethylphenyl)-2-(4-methoxyphenyl)quinoline-4-carboxylic acid
• 8-(4-butylphenyl)-2-(4-methoxyphenyl)quinoline-4-carboxylic acid
• 8-(4-(tert-butyl)phenyl)-2-(4-methoxyphenyl)quinoline-4-carboxylic acid
• 8-(4-acetylphenyl)-2-(4-methoxyphenyl)quinoline-4-carboxylic acid
• 8-(3,4-dichlorophenyl)-2-(4-methoxyphenyl)quinoline-4-carboxylic acid
• 2-(4-methoxyphenyl)-8-(3-(trifluoromethoxy)phenyl)quinoline-4-carboxylic acid
• 2-(4-methoxyphenyl)-8-(4-propoxyphenyl)quinoline-4-carboxylic acid
• 2-(4-methoxyphenyl)-8-(3,4,5-trimethoxyphenyl)quinoline-4-carboxylic acid
• 2-(4-methoxyphenyl)-8-(4-morpholinophenyl)quinoline-4-carboxylic acid and pharmaceutically acceptable salts thereof.

15. A pharmaceutical composition comprising a compound according to any one of claim 1 to 6 and at least one pharmaceutically acceptable excipient for use in the treatment and/or prevention of viral infections.
